# EUROPEAN PATENT APPLICATION

(11) **EP 4 674 341 A1**
(43) Date of publication of application: **07.01.2026**
(21) Application number: 24763279.7
(22) Date of filing: 01.03.2024
(51) Int. Cl.: A61B 5/00, F16M 11/04

(54) **MONITORING DEVICE AND MEDICAL DEVICE**

(30) Priority: 01.03.2023 CN 202310233270
(71) Applicant: Shenzhen Mindray Bio-Medical Electronics Co., Ltd., Shenzhen, Guangdong 518057 (CN)
(72) Inventor: LIAO, Xing, Shenzhen, Guangdong 518057 (CN); WU, Dingwei, Shenzhen, Guangdong 518057 (CN); WU, Shubing, Shenzhen, Guangdong 518057 (CN); ZHONG, Panxi, Shenzhen, Guangdong 518057 (CN); ZOU, Huan, Shenzhen, Guangdong 518057 (CN); LU, Yi, Shenzhen, Guangdong 518057 (CN); CHEN, Dong, Shenzhen, Guangdong 518057 (CN); WU, Bingzhe, Shenzhen, Guangdong 518057 (CN); XU, Bin, Shenzhen, Guangdong 518057 (CN); WANG, Xianghui, Shenzhen, Guangdong 518057 (CN); HE, Lijuan, Shenzhen, Guangdong 518057 (CN); DENG, Nanfang, Shenzhen, Guangdong 518057 (CN); LI, Yangfei, Shenzhen, Guangdong 518057 (CN); XU, Li, Shenzhen, Guangdong 518057 (CN)
(74) Representative: KIPA AB
(86) International application number: PCT/CN2024/079762
(87) International publication number: WO 2024/179599

(57) **Abstract**

Provided in the embodiments of the present application are a monitoring device and a medical device. The monitoring device comprises a main control board, a device main body, a screen assembly and a connection assembly, wherein the device main body comprises a casing; the screen assembly comprises a mounting housing and a screen arranged on the mounting housing; and the screen assembly is movably connected to the device main body by means of the connection assembly, such that the screen assembly can be deflected from a first position to a second position relative to the device main body, and at least a portion of the screen assembly gradually moves away or approaches the device main body when the screen assembly is deflected from the first position to the second position.

## Description

### CROSS-REFERENCE OF RELATED APPLICATIONS

The application is based on a Chinese patent application No. 202310233270.6, filed on March 1, 2023, and claims priority to said Chinese patent application; the contents of which are incorporated herein by reference in their entirety.

### TECHNICAL FIELD

The disclosure relates to a technical field of medical device, and in particular to a monitoring device and a medical device.

### BACKGROUND

Monitor is a medical device that is widely used in different departments of major hospitals. The monitor is mostly used to monitor critically ill and sub-critically ill patients. Medical staff read physiological parameters of patient, which parameters are fed back by the monitor to understand vital signs of patient in real time. Therefore, the monitor needs to provide medical staff with clear data display in different clinical usage scenarios.

However, in the related technology, when the monitor is placed at a high place (such as a device, for example a stand of a light, patient support, or tower), it is easily affected by other external factors, such as light. Medical staff may not be able to clearly read data content displayed by the monitor, which may easily delay a determination for vital signs of patient or make an incorrect determination about vital signs of patient, thereby indirectly causing some irreversible clinical accidents.

### SUMMARY

In view of this, embodiments of this disclosure are intended to provide a monitoring device and a medical device that are convenient to read data.

To achieve the above-mentioned purpose, an embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a mounting housing and a screen which is arranged at the mounting housing;
a connection assembly, wherein the screen assembly is in movable connection with the device body through the connection assembly, so as to enable the screen assembly to be tilted from a first position to a second position relative to the device body; wherein when the screen assembly is tilted from the first position to the second position, at least a portion of the screen assembly gradually moves away from or close to the device body.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a mounting housing and a screen which is arranged at the mounting housing;
a connection assembly, wherein the screen assembly is in movable connection with the device body through the connection assembly, so as to enable the screen assembly to be tilted downward from a first position to a second position relative to the device body to exhibit a posture with an angle of depression; wherein when the screen assembly is tilted downward from the first position to the second position, an upper portion of the screen assembly gradually moves away from the device body or a lower portion of the screen assembly gradually moves close to the device body.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a mounting housing and a screen which is arranged at the mounting housing;
a connection assembly, wherein the screen assembly is in movable connection with the device housing through the connection assembly, so as to enable the screen assembly to be tilted downward from an initial position relative to the device body to form a posture with an angle of depression; wherein when the screen assembly is tilted downward from the initial position, the entirety of the monitoring device maintains stability.

An embodiment of this disclosure provides a medical device, including:
a device body, which includes a device housing;
a screen assembly, which includes a mounting housing and a screen which is arranged at the mounting housing; wherein the screen assembly is in movable connection with the device body and is capable of being tilted downward from a first position to a second position relative to the device body to exhibit a posture with an angle of depression, wherein when the screen assembly is tilted from the first position to the second position, an upper edge of the screen assembly gradually moves away from the device body or a lower edge of the screen assembly gradually moves away from the device body, so as to form a gap region between the screen assembly and the device body;
a cover housing, which is located between the device body and the screen assembly, so as to cover the gap region.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a mounting housing and a screen which is arranged at the mounting housing; wherein the screen assembly is in movable connection with the device body and is capable of being tilted from a first position to a second position relative to the device body; wherein when the screen assembly is tilted from the first position to the second position, at least a portion of the screen assembly gradually moves away from or close to the device body;
a position-restricting structure, which is configured to restrict the screen assembly to the first position, the second position, or at least one intermediate position between the first position and the second position, so as to restrict the screen assembly from rotating relative to the device body.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data; and
a device body, which includes a device housing;
wherein, the monitoring device is provided with a convex portion which protrudes forward from a bottom side of the device housing;
a screen assembly, which includes a mounting housing and a screen which is arranged at the mounting housing; wherein the screen assembly is in movable connection with the device body and is capable of being tilted from a first position to a second position relative to the device body;
wherein, a lower edge of the screen assembly is located above the convex portion.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a support portion, a mounting housing, and a screen which is arranged at the mounting housing; wherein the screen assembly is in movable connection with the device body and is capable of being tilted downward from an initial position to an extreme position relative to the device body to form a posture with an angle of depression; wherein, the support portion supports the screen assembly at at least one position, while the screen assembly is tilted downward from the initial position to the extreme position.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a mounting housing and a screen which is arranged at the mounting housing;
a connection assembly, which is mounted on a front side of the device housing; wherein the screen assembly is in movable connection with the device body through the connection assembly, so as to enable the screen assembly to be tilted downward from an initial position to an extreme position relative to the device body;
wherein, while the screen assembly is tilted downward from the initial position to the extreme position, a distance between an upper edge of the screen assembly and the device body gradually increases, and a distance between a lower edge of the screen assembly and the device body gradually decreases; and wherein when the screen assembly is tilted to the extreme position, the screen assembly is in contact with the device housing or a gap is formed between the screen assembly and the device housing.

An embodiment of this disclosure provides a medical device, including:
a device body, which includes a device housing;
a screen assembly, which includes a mounting housing and a screen which is arranged at the mounting housing;
a connection assembly, wherein the screen assembly is in movable connection with the device body through the connection assembly; wherein the connection assembly includes a first slidable portion and a second slidable portion which are slidably fitted with each other; wherein the first slidable portion is arranged at the device housing and the second slidable portion is arranged at the mounting housing, so as to enable the screen assembly to be tilted relative to the device body for adjusting an angle between the screen assembly and a horizontal plane.

An embodiment of this disclosure provides a monitoring device, which is configured to obtain and process physiological parameter data of a patient acquired by a parameter sensor, and display processed physiological parameter data of the patient, wherein the monitoring device includes:
a main control board, which is configured to obtain the physiological parameter data of the patient acquired by the parameter sensor and process the physiological parameter data;
a device body, which includes a device housing;
a screen assembly, which includes a mounting housing and a screen which is arranged at the mounting housing;
a connection assembly, wherein the screen assembly is in movable connection with the device body through the connection assembly, so as to enable the screen assembly to be tilted from a first position to a second position relative to the device body; wherein when the screen assembly is tilted from the first position to the second position, at least a portion of the screen assembly gradually moves away from or close to the device body, wherein tilting of the screen assembly satisfies one of following conditions:
   a movement trajectory of a center of mass of the screen assembly is an irregular circular movement;
   the screen assembly performs a circular movement around two or more points;
   the screen assembly performs a circular movement around two or more axes.

Embodiments of this disclosure provide a monitoring device and a medical device. For the monitoring device, a screen assembly is in movable connection with a device body and is capable of being tilted from a first position to a second position relative to the device body. Therefore, medical staff can adjust an angle of the screen assembly as needed to clearly read physiological parameter data displayed on a screen, thereby better preventing medical staff from delaying a determination for vital signs of patient or making an incorrect determination on vital signs of patient, thereby reducing a probability of some clinical accidents.

### BRIEF DESCRIPTION OF THE DRAWINGS

FIG. 1 is a structural diagram for a first monitoring device at a first position according to an embodiment of this disclosure.
FIG. 2 is a structural diagram for a monitoring device shown in FIG. 1 from another perspective.
FIG. 3 is a structural diagram for a monitoring device shown in FIG. 1 at a second position.
FIG. 4 is a structural diagram for a monitoring device shown in FIG. 3 from another perspective.
FIG. 5 is a schematic diagram for a support region of a monitoring device according to an embodiment of this disclosure.
FIG. 6 is a schematic diagram for another support region of a monitoring device according to an embodiment of this disclosure.
FIG. 7 is a structural diagram for a second monitoring device at a first position according to an embodiment of this disclosure.
FIG. 8 is a structural diagram for a connection assembly shown in FIG. 7.
FIG. 9 is a structural diagram for a monitoring device shown in FIG. 7 at a second position.
FIG. 10 is a structural diagram for a third monitoring device at a second position according to an embodiment of this disclosure.
FIG. 11 is a structural diagram for a connection assembly shown in FIG. 10.
FIG. 12 is a partially structural diagram for a fourth monitoring device according to an embodiment of this disclosure.
FIG. 13 is a cross-sectional view for a fifth monitoring device at a first position according to an embodiment of this disclosure.
FIG. 14 is a partially enlarged view for point A in FIG. 13.
FIG. 15 is a partially enlarged view for point B in FIG. 13.
FIG. 16 is a structural diagram for a monitoring device shown in FIG. 13 at a second position.
FIG. 17 is a partially enlarged view for point C in FIG. 16.
FIG. 18 is a partially enlarged view for point D in FIG. 16.
FIG. 19 is a cross-sectional view for a sixth monitoring device at a first position according to an embodiment of this disclosure.
FIG. 21 is a schematic diagram for a connection relationship between a device housing and a connection assembly of a seventh monitoring device according to an embodiment of this disclosure.
FIG. 22 is a schematic diagram for a connection relationship between a connection assembly and a mounting housing shown in FIG. 21.
FIG. 23 is a structural diagram for a connection assembly shown in FIG. 21.
FIG. 24 is an exploded view for a connection assembly shown in FIG. 23.
FIG. 25 is a cross-sectional view for an eighth monitoring device at a first position according to an embodiment of this disclosure.
FIG. 26 is a partially enlarged view for point E in FIG. 25.
FIG. 27 is a structural diagram for a monitoring device shown in FIG. 25 at a second position.
FIG. 28 is a cross-sectional view for a monitoring device shown in FIG. 27.
FIG. 29 is a partially enlarged view for point F in FIG. 28.
FIG. 30 is a cross-sectional view for a ninth monitoring device at a first position according to an embodiment of this disclosure.
FIG. 31 is a cross-sectional view for a monitoring device shown in FIG. 30 at a second position.
FIG. 32 is a cross-sectional view for a tenth monitoring device at the first position according to the embodiment of this disclosure.
FIG. 33 is a cross-sectional view for a monitoring device shown in FIG. 32 at a second position.
FIG. 34 is a structural diagram for an eleventh monitoring device at a first position according to an embodiment of this disclosure.
FIG. 35 is a structural diagram for a monitoring device shown in FIG. 34 at a second position.
FIG. 36 is a cross-sectional view for a twelfth monitoring device at a first position according to an embodiment of this disclosure.
FIG. 37 is a cross-sectional view for a monitoring device shown in FIG. 36 at a second position.
FIG. 38 is a cross-sectional view for a thirteenth monitoring device at a first position according to an embodiment of this disclosure.
FIG. 39 is a cross-sectional view for a monitoring device shown in FIG. 38 at a second position.
FIG. 40 is a cross-sectional view for a fourteenth monitoring device according to an embodiment of this disclosure at a first position.
FIG. 41 is a cross-sectional view for a monitoring device shown in FIG. 40 at a second position.
FIG. 42 is a structural diagram for a fifteenth monitoring device at a second position according to an embodiment of this disclosure.
FIG. 43 is a structural diagram for a sixteenth monitoring device at a first position according to an embodiment of this disclosure.
FIG. 44 is a structural diagram for a monitoring device shown in FIG. 43 at a second position.
FIG. 45 is a structural diagram for a seventeenth monitoring device at a first position according to an embodiment of this disclosure.
FIG. 46 is a structural diagram for a monitoring device shown in FIG. 45 at a second position.
FIG. 47 is a structural diagram for an eighteenth monitoring device at a second position according to an embodiment of this disclosure.
FIG. 48 is a cross-sectional view for a nineteenth monitoring device at a first position according to an embodiment of this disclosure.
FIG. 49 is a cross-sectional view for a monitoring device shown in FIG. 48 at a second position.
FIG. 50 is a structural diagram for a twentieth monitoring device at a first position according to an embodiment of this disclosure.
FIG. 51 is a structural diagram for a monitoring device shown in FIG. 50 at a second position.
FIG. 52 is a partially structural diagram for a twenty-first monitoring device at a first position according to an embodiment of this disclosure.
FIG. 53 is a partially structural diagram for a monitoring device shown in FIG. 52 at a second position.

### DESCRIPTION OF THE PREFERRED EMBODIMENTS

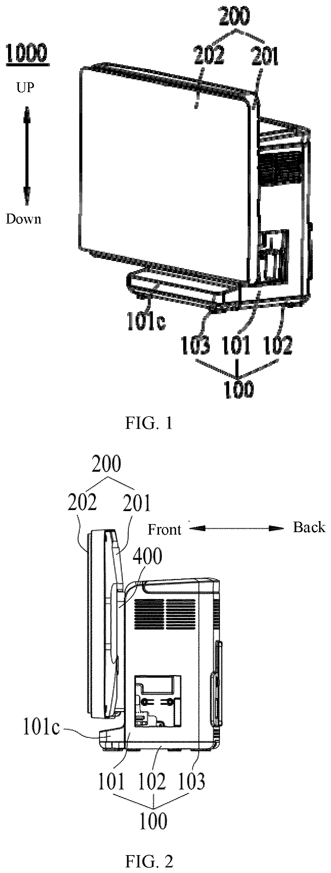

In description of embodiments of this disclosure, it should be noted that directions or positional relationships indicated by terms "up" and "low" are based on directions or positional relationships shown in FIG. 1, and directions or positional relationships indicated by terms "front" and "back" are based on directions or positional relationships shown in FIG. 2, wherein "top" refers to an upper direction as shown in FIG. 1, and "bottom" refers to a lower direction as shown in FIG. 1. These directions terms are only for a convenience of describing the embodiments of this disclosure and simplifying the description, and do not indicate or imply that devices or elements referred to must have a specific direction, be constructed and operated in a specific direction, and therefore should not be understood as limitations on the embodiments of this disclosure.

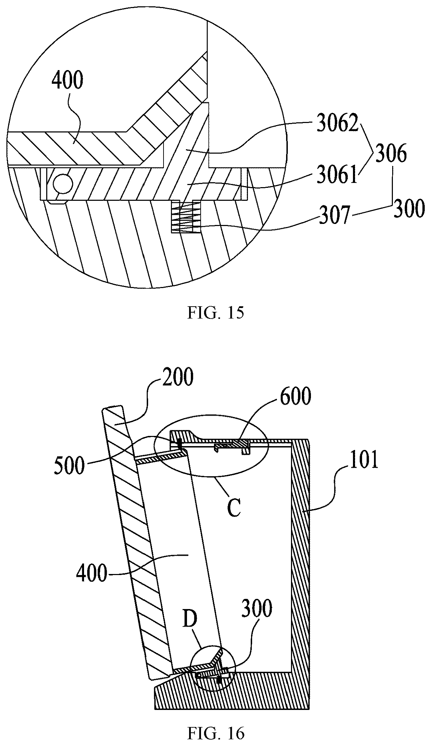

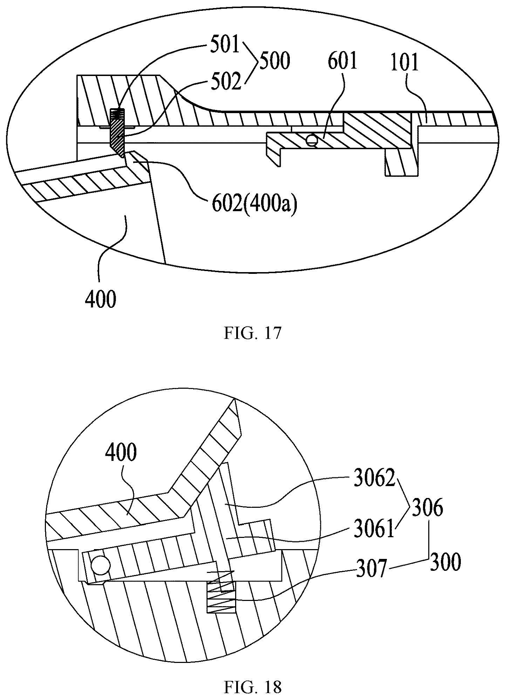

An embodiment of this disclosure provides a medical device 1000, please refer to FIGS. 1 to 51, the medical device 1000 includes a device body 100 and a screen assembly 200, wherein the device body 100 includes a device housing 101. The screen assembly 200 includes a mounting housing 201 and a screen 202 arranged at the mounting housing 201.

The medical device 1000 may be a monitoring device, for example, an integrated monitor through which physiological parameter data of a patient is acquired; or a plug-in monitor to which a parameter module may be plugged and from which physiological parameter data of a patient is acquired.

An embodiment of this disclosure is described by taking the medical device 1000 as a monitoring device 1000 for example.

The monitoring device 1000 is configured to obtain and process physiological parameter data of a patient, which data are acquired by a parameter sensor, and display processed physiological parameter data of the patient.

In one embodiment, the monitoring device 1000 is an integrated monitor. A main control board and a parameter sensor interface, which are connected with the main control board, are provided inside the device housing 101 of the monitoring device 1000. The parameter sensor interface is configured to connect a parameter sensor to obtain physiological parameter data of a patient, which data are acquired by the parameter sensor.

The main control board may be composed of two independent control boards, namely a mainboard and a parameter board. For example, the parameter board is in electrical connection with the mainboard, and the parameter sensor interface is connected with the parameter board. The main control board may also be a control board having functions of a mainboard and a parameter board, and the parameter sensor interface is directly connected with the main control board. In addition, the main control board and the parameter sensor interface can also be combined together. The parameter sensor interface may also be arranged inside the mounting housing 201 and connected with the main control board via a cable.

In an optional solution, the monitoring device 1000 is an integrated monitor, and the main control board may include two independent control boards, namely a mainboard and a parameter board. The mainboard is arranged inside one of the mounting housing 201 and the device housing 101, and the parameter board is arranged inside the other one of the mounting housing 201 and the device housing 101, and the mainboard and the parameter board are interconnected by a cable. Furthermore, when the mainboard is arranged inside one of the mounting housing 201 and the device housing 101, the parameter sensor interface can be arranged inside the other one of the mounting housing 201 and the device housing 101, and the mainboard and the parameter board are interconnected by a cable; the parameter sensor interface can also be arranged inside a same one of the mounting housing 201 and the device housing 101 as the mainboard. The main control board can also be a control board with functions of a mainboard and a parameter board.

In an optional solution, the monitoring device 1000 is an integrated monitor, and the main control board may include a mainboard and a parameter board that are independent of each other or integrated with each other, and both of the mainboard and the parameter board are arranged inside the mounting housing 201. The parameter sensor interface may be arranged inside the mounting housing 201, or may be arranged inside the device housing 101 and connected with the main control board via a cable.

In another embodiment, the monitor 1000 is a plug-in monitor, and its main control board may include a mainboard and a communication interface board. Similar to the above embodiment, arrangement positions of the mainboard and the communication interface board can have three different combinations: both being arranged inside the device housing 101; both being arranged inside the mounting housing 201; one being arranged inside the device housing 101 and the other one being arranged inside the mounting housing 201, and both boards being connected with each other via a cable.

A processor is provided on the main control board of the monitoring device 1000, and the processor is configured to obtain physiological parameter data of a patient acquired by the parameter sensor, and process the physiological parameter data. When the monitoring device 1000 is an integrated monitor, the parameter sensor interface is connected with the processor, and the parameter sensor interface is connected with the parameter sensor to obtain the physiological parameter data of the patient acquired by the parameter sensor; the processor is configured to obtain the physiological parameter data of the patient acquired by the parameter sensor through the parameter sensor interface, and process the physiological parameter data. When the monitoring device 1000 is a plug-in monitor, the communication interface board is connected with the processor, and the communication interface board is configured to obtain, from an external plug-in, the physiological parameter data of the patient acquired by the parameter sensor; the processor is configured to obtain the physiological parameter data of the patient acquired by the parameter sensor and then transmitted by the communication interface board, and process the physiological parameter data.

A screen 202 of the monitoring device 1000 is configured to display processed physiological parameter data of the patient.

Please refer to FIGS. 1 to 4. The screen assembly 200 is in movable connection with the device body 100, so as to enable the screen assembly 200 to be tilted from a first position to a second position relative to the device body 100. When the screen assembly 200 is tilted from the first position to the second position, at least a portion of the screen assembly 200 gradually moves away from or close to the device body 100.

Tilting means that the screen assembly 200 at least has a type of a movement manner of a rotation, while the screen assembly 200 moves from the first position to the second position, that is, an angle of the screen assembly 200 changes, while the screen assembly 200 moves from the first position to the second position. The screen assembly 200 can be moved from the first position to the second position only by a rotation movement, which is equivalent to that while the screen assembly 200 moves from the first position to the second position, a position of a tilting axis of the screen assembly 200 remains unchanged. The screen assembly 200 can also be moved from the first position to the second position by combining a rotation with other type(s) of movement, which is equivalent to that while the screen assembly 200 moves from the first position to the second position, a position of a tilting axis of the screen assembly 200 changes.

For example, the screen assembly 200 can be moved from the first position to the second position by combining a rotation movement and a linear movement.

For example, tilting of the screen assembly 200 may satisfy one of following conditions: a movement trajectory of a center of mass of the screen assembly 200 is an irregular circular movement; the screen assembly 200 performs a circular movement around two or more points; the screen assembly 200 performs a circular movement around two or more axes.

The first position and the second position are two positions of the screen assembly 200 which change before and after the screen assembly 200 is tilted. In some embodiments, the first position may be an initial position, and the second position may be an extreme position, when a tilting angle reaches a maximum. In other embodiments, the first position may not be an initial position, and the second position may not be an extreme position.

The monitoring device 1000 can be arranged with multiple adjustable gears, wherein one adjustable gear corresponds to a first position, another adjustable gear corresponds to a second position, and at least a further adjustable gear corresponds to at least one intermediate position. That is, the screen assembly 200 can be tilted from the first position to the second position by switching from one gear to another gear, or the screen assembly 200 can also be tilted from the first position to the second position by a continuous rotation movement.

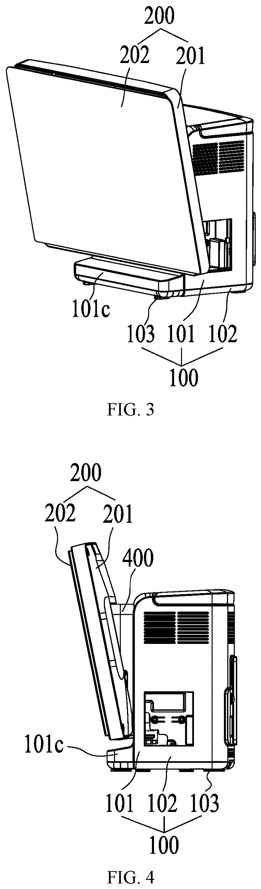

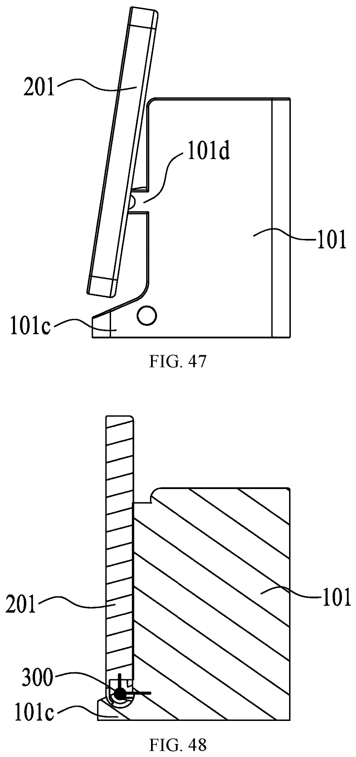

At least a portion of the screen assembly 200 gradually moving away from or moving close to the device body 100, means that according to different design forms, the screen assembly 200 can be tilted in a direction away from the device body 100, or in a direction towards the device body 100. For example, the screen assembly 200 can be tilted downward as shown in FIG. 3 or upward as shown in FIG. 47 (according to different design requirements, the screen assembly 200 can be tilted only downward, only upward, or both downward and upward), or can be tilted leftward or rightward (according to different design requirements, the screen assembly 200 can be tilted only leftward, only rightward, or both to leftward and rightward).

Exemplarily, referring to FIGS. 3 and 4, the screen assembly 200 is capable of being tilted downward from a first position to a second position relative to the device body 100, so as to exhibit a posture with an angle of depression. When the screen assembly 200 is tilted downward from the first position to the second position, an upper portion of the screen assembly 200 gradually moves away from the device body 100.

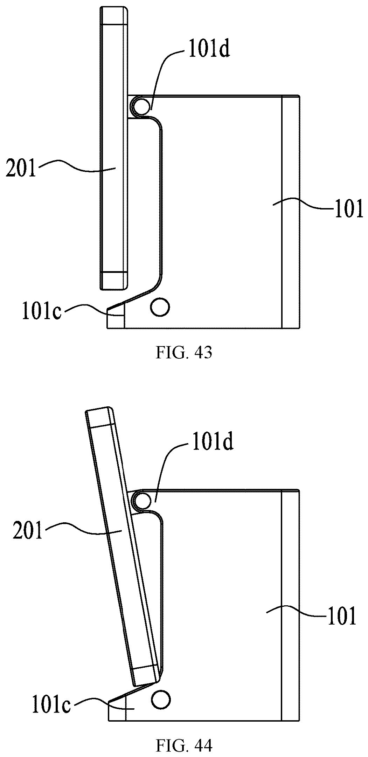

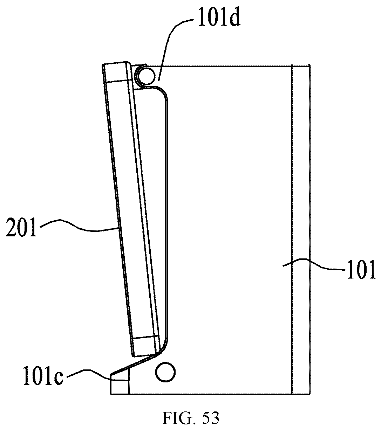

Please refer to FIGS. 43, 44, 52 and 53. When the screen assembly 200 is tilted downward from a first position to a second position, a lower portion of the screen assembly 200 gradually moves close to the device body 100, that is, a tilting axis of the screen assembly 200 is not located at a bottom of the screen assembly 200.

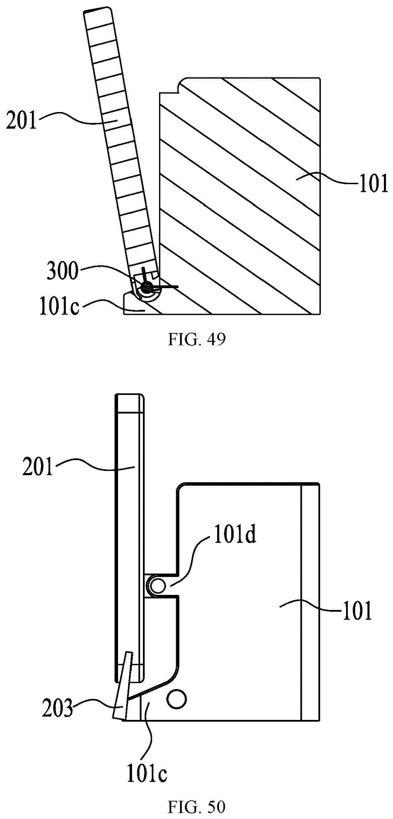

In other embodiments, a lower portion of the screen assembly 200 may not gradually move close to the device body 100. For example, a tilting axis of the screen assembly 200 may be arranged at a bottom of the screen assembly 200 as shown in FIGS. 48 and 49.

The screen assembly 200 can be in movable connection with the device body 100 via a connection assembly 300, so as to enable the screen assembly 200 to be tilted from a first position to a second position relative to the device body 100.

The connection assembly 300 can be arranged independently, or in other words, the connection assembly 300 can be connected with the screen assembly 200 and the device body 100 respectively, and the connection assembly 300 can also be a portion of the screen assembly 200 or a portion of the device body 100.

In some embodiments, the screen assembly 200 may not be in movable connection with the device body 100 through the connection assembly 300.

The screen assembly 200 shown in FIG. 1 is in an upright posture at the first position. At the first position, the screen assembly 200 is in a vertical state, and an angle between the screen assembly 200 and a top surface of the device body 100 is 80-100 degrees, for example, the screen assembly 200 can be substantially perpendicular to the top surface of the device body 100.

In other embodiments, the screen assembly 200 may be in a posture with an angle of elevation at the first position, or in a posture with an angle of depression, which angle is less than that at the second position.

For the monitoring device 1000, the screen assembly 200 is in movable connection with the device body 100 and is capable of being tilted from a first position to a second position relative to the device body 100. Therefore, medical staff can adjust the angle of the screen assembly 200 as needed to facilitate clear reading of the physiological parameter data displayed on the screen 202, thereby better preventing medical staff from delaying a determination of the vital signs of patient or making an incorrect determination on the vital signs of patient, thereby reducing a probability of some clinical accidents.

In addition, for the monitoring device 1000, in which the screen assembly 200 is capable of being tilted downward from a first position to a second position relative to the device body 100 to form a posture with an angle of depression, when the monitoring device 1000 is placed at a high place (such as a device, for example a stand of a light, patient support, or tower), the screen assembly 200 can form an angle of depression by tilting downward, so as to reduce an influence of other external factors, such as light, and make it easier for medical staff to clearly read the physiological parameter data displayed on the screen 202.

In one embodiment, referring to FIG. 3 and FIG. 4, the screen assembly 200 is capable of being tilted downward from a first position relative to the device body 100, so as to form a posture with an angle of depression. When the screen assembly 200 is tilted downward from the first position, the entirety of the monitoring device 1000 maintains stability.

That is to say, while the screen assembly 200 is tilted downward from the first position and after the screen assembly 200 forms a posture with an angle of depression, the entirety of the monitoring device 1000 maintains stability, and the monitoring device 1000 will not become unstable and fall due to a shift of a center of gravity.

For example, maintaining stability may mean that the monitoring device 1000 does not fall forward, while the screen assembly 200 is tilted downward from the first position.

For example, maintaining stability may also mean that a bottom surface of the monitoring device 1000, that is in contact with a support surface which supports the device body 100, is always in contact with the support surface.

The support surface which supports the device body 100 refers to a support surface on a support object, such as a ground, a desktop or a bracket on which the monitoring device 1000 is placed.

That is to say, after the monitoring device 1000 is placed on the support object, at least a portion of the bottom surface of the monitoring device 1000 will be in contact with the support surface on the support object, and the bottom surface of the monitoring device 1000, which is in contact with the support surface, always maintains in contact with the support surface, and will not be separated from the support surface.

For example, maintaining stability may also mean that when the screen assembly 200 is tilted downward from the first position, the monitoring device 1000 does not fall forward, and meanwhile the bottom surface of the monitoring device 1000, which is in contact with the support surface, is not separated from the support surface.

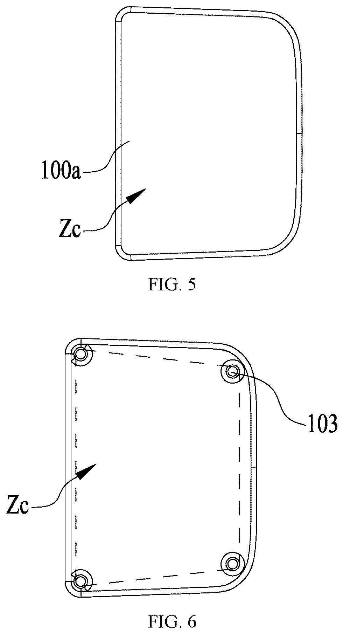

In one embodiment, please refer to FIGS. 5 and 6. While the screen assembly 200 is tilted downward from the first position, a projection of a center of gravity of the monitoring device 1000 on a horizontal plane is always located within a projection range of the support region Zc, which support region supports the device body 100 on said horizontal plane.

The support region Zc refers to a region on the device body 100, which region plays a support role. At least a portion of the device body 100, which portion is located inside the support region, needs to be in contact with the support surface.

For example, referring to FIG. 5, a continuous support contact surface 100 a is constructed on the bottom surface of the device body 100, and a region which is enclosed by an outer contour of the support contact surface 100 a is the support region Zc. The continuous support contact surface 100a means that a bottom of the device body 100 is provided with a plane, which is in contact with the support surface. For example, if at least a portion of a bottom surface of the device housing 101 is in contact with the support surface, then a region of the bottom surface of the device housing 101, which region is in contact with the support surface, is the support contact surface 100a. If the device body 100 is provided with a base 102, and at least a portion of a bottom surface of the base 102 is in contact with the support surface, then a region of the bottom surface of the base 102, which region is in contact with the support surface, is the support contact surface 100a.

For example, please refer to FIG. 6. If a plurality of support legs 103 are arranged at a bottom of the device housing 101, outer contours of bottom surfaces of each leg 103 are connected in sequence by straight lines, and a largest region enclosed is the support region Zc (i.e., a region enclosed by dotted lines in FIG. 6). That is to say, except for a plane on which each support leg 103 is in contact with the support surface, a maximum region, which is enclosed by the outer contours of the bottom surfaces of each support leg 103 through straight lines, belongs to the support region Zc, which is equivalent to that, in the support region Zc, only the bottom surface of the support leg 103 is in contact with the support surface, and other portions of the device body 100, which portions are located inside the support region, are hardly in contact with the support surface.

In addition, it should be noted that the support leg 103 at the bottom of the device housing 101 can be directly arranged at the device housing 101. If the device body 100 is provided with a base 102, the support leg 103 is arranged at the base 102.

In order to avoid the monitoring device 1000 from becoming unstable and tipping over due to a shift of a center of gravity of the monitoring device 1000, while the screen assembly 200 is tilted downward and when the screen assembly 200 is tilted to the second position, a maximum of the angle of depression of the screen assembly 200 can be 45°, that is, a downward rotation angle of the screen assembly 200 does not exceed 45°.

More preferably, a maximum of the angle of depression of the screen assembly 200 can be 20°.

In addition, when the monitoring device 1000 is placed at a high place, an angle of depression of the screen assembly 200 is generally within 10°, which is sufficient for medical staff to clearly read the physiological parameter data displayed on the screen 202. Therefore, under normal circumstances, a maximum of the angle of depression of the screen assembly 200 can be 10°.

In one embodiment, referring to FIG. 1 and FIG. 43 to FIG. 46, a portion of a bottom side of the device housing 101 may protrude from a front side of the device housing 101, so as to form a convex portion 101c, and the screen assembly 200 is arranged on an upper side of the convex portion 101c.

The convex portion 101c mainly plays a stabilization role, especially for the screen assembly 200 that tilts downward, which may effectively reduce a risk of the monitoring device 1000 for losing its center of gravity due to downward tilting of the screen assembly 200.

In one embodiment, referring to FIGS. 43 to 46, a lower edge of the screen assembly 200 may be located above the convex portion 101c, that is, the lower edge of the screen assembly 200 will not be in contact with the convex portion 101c.

For the monitoring device 1000, in which the screen assembly 200 is tilted downward to the second position, for example, an upper surface of the convex portion 101c may have a shape which is capable of accommodating with a movement of the lower edge of the screen assembly 200, while the screen assembly 200 is tilted from the first position to the second position.

Exemplarily, referring to FIGS. 43 to 46, an upper surface of the convex portion 101c may also be inclined downward from a rear side to a front side.

In one embodiment, referring to FIG. 2 and FIG. 4, the screen assembly 200 is tilted about a tilting axis, which extends horizontally. The screen assembly 200 may be tilted downward from a first position to a second position about the tilting axis, or may be tilted upward from the first position to the second position about the tilting axis. A size of a space formed between the screen assembly 200 and the convex portion 101c changes with tilting of the screen assembly 200. At least when the screen assembly 200 is at the first position or the second position, a cleanable space can be formed between the screen assembly 200 and the convex portion 101c. The cleanable space refers to a space formed between the screen assembly 200 and the convex portion 101c, which space is large enough to satisfy a general cleaning requirement, that is, at least some cleaning tools or human fingers can be inserted into the space formed between the screen assembly 200 and the convex portion 101c, so as to clean the space.

Since the screen assembly 200 is arranged at the upper side of the convex portion 101c, a certain space is formed between the screen assembly 200 and the convex portion 101c. While the screen assembly 200 is tilted, a size of the space between the screen assembly 200 and the convex portion 101c will also change with tilting of the screen assembly 200. Therefore, in order to prevent dust from accumulating in the space between the screen assembly 200 and the convex portion 101c, a cleanable space can be formed between the convex portion 101c and the screen assembly 200, at least when the screen assembly 200 is at the first position or the second position.

In one embodiment, referring to FIG. 1, a bottom of the device housing 101 may also be provided with a base 102 to achieve stability. For the monitoring device 1000 provided with a base 102, a bottom side of the device housing 101 may be provided with a convex portion 101c, or may not be provided with the convex portion 101c.

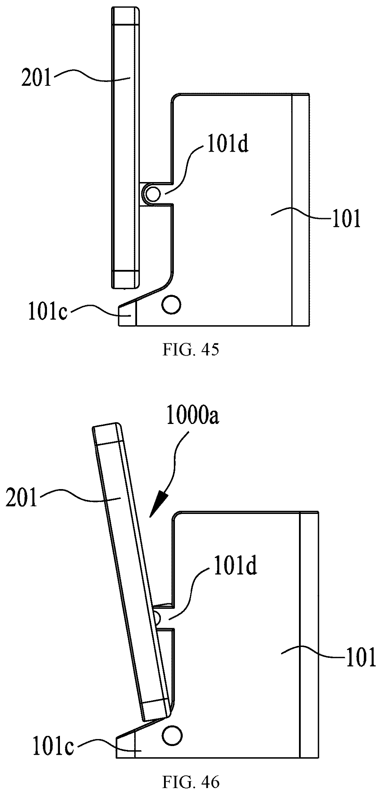

In one embodiment, please refer to FIGS. 45 and 46, a distance between a tilting axis of the screen assembly 200 relative to the device body 100 and a horizontal center line of the monitoring device 1000 is less than a distance between said tilting axis and a top end surface of the monitoring device 1000, and a distance between said tilting axis and a bottom end surface of the monitoring device 1000.

That is to say, a tilting axis of the screen assembly 200 is approximately located inside a middle of the monitoring device 1000, and the tilting axis is neither adjacent to a top end surface of the monitoring device 1000 nor adjacent to a bottom end surface of the monitoring device 1000. The tilting axis is approximately located inside a middle of the monitoring device 1000, which can ensure that a center of gravity of the monitoring device 1000 hardly shifts before and after the screen assembly 200 is tilted, thereby improving an overall stability of the monitoring device 1000.

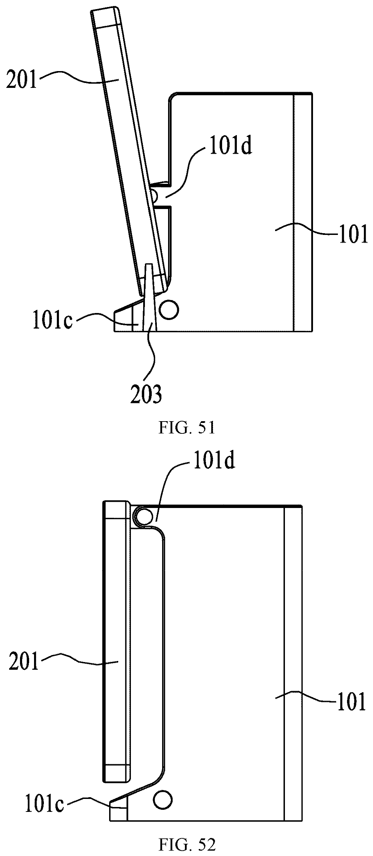

In one embodiment, please refer to FIGS. 50 and 51, the screen assembly 200 can also be provided with a support portion 203, and when the screen assembly 200 is tilted downward from a first position to a second position, the support portion 203 supports the screen assembly 200. That is to say, before the screen assembly 200 is tilted to the second position, the support portion 203 is not in contact with the support surface. After the screen assembly 200 is tilted to the second position, the support portion 203 is in contact with the support surface to support the screen assembly 200 together with the device body 100, thereby also playing a stabilization role. In one embodiment, the support portions 203 may be arranged on both sides of the screen assembly 200 and extend downward from both sides of the screen assembly 200. After the screen assembly 200 is tilted to the second position, when the support portion 203 is in contact with the support surface, the support portion 203 may be perpendicular to the support surface.

The support portion 203 may be independently provided or integrally formed with the screen assembly 200.

In some embodiments, for a monitoring device in which the screen assembly 200 is capable of being tilted downward from an initial position to an extreme position relative to the device body 100, so as to form a posture with an angle of depression, the support portion 203 is also capable of supporting the screen assembly 200 at at least one position, while the screen assembly 200 is tilted downward from the initial position to the extreme position, that is, the support portion 203 is capable of supporting the screen assembly 200 at the initial position, or the extreme position, or an intermediate position between the initial position and the extreme position.

In addition, the support portion 203 is capable of supporting the screen assembly 200 in a vertical position, and not supporting the screen assembly 200 after tilting.

In one embodiment, please refer to FIGS. 2 and 4, the monitoring device 1000 can be provided with a cover housing 400. When the screen assembly 200 is tilted relative to the device body 100, a gap region 1000a is formed between the screen assembly 200 and the device body 100 (please refer to FIG. 46). The cover housing 400 can be arranged between the device housing 101 and the mounting housing 201, or at one of the device housing 101, the mounting housing 201 and the connection assembly 300, so as to cover the gap region 1000a, which gap region is formed between the device body 100 and the screen assembly 200 due to tilting of the screen assembly 200 relative to the device body 100.

Specifically, the cover housing 400 can be an independent member, that is, the cover housing 400 can be mounted between the device housing 101 and the mounting housing 201 by assembly. The cover housing 400 can also be directly arranged at the device housing 101, or directly arranged at the mounting housing 201, or directly arranged at the connection assembly 300. For example, the cover housing 400 can be fixed to the device housing 101, the mounting housing 201 or the connection assembly 300 by snap-fitting, plug-in, welding, bonding, fastening with fasteners such as screws, etc. The cover housing 400 can also be integrally formed with the device housing 101 or the mounting housing 201 or the connection assembly 300, which is equivalent to that the cover housing 400 constituting a portion of a structure of the device body 100, of the screen assembly 200 or of the connection assembly 300.

In some embodiments, the screen assembly 200 can also be in movable connection with the device body 100 via the cover housing 400, so as to enable the screen assembly 200 to be tilted from a first position to a second position relative to the device body 100. In other words, the cover housing 400 can be used to drive the screen assembly 200 to tilt without requiring an arrangement of a connection assembly 300.

The cover housing 400 may be a hard housing made of a rigid material, or a soft housing made of a flexible material. When being made of a flexible material, the cover housing 400 may be an accordion-like pleated structure.

In another embodiment, a connection assembly 300 may be a cover housing 400, that is, the connection assembly 300 and the cover housing 400 are integrated with each other.

The gap region 1000a is a gradually changed space, which is formed between the screen assembly 200 and the device body 100, while the screen assembly 200 is tilted relative to the device body 100.

Exemplarily, referring to FIG. 46, while the screen assembly 200 is tilted downward from a first position to a second position, an upper edge of the screen assembly 200 gradually moves away from the device body 100, and a gradually increased gap region 1000a is formed between the screen assembly 200 and the device body 100.

Please refer to FIG. 2 and FIG. 4, the cover housing 400 can always cover the gap region 1000a, that is, while the screen assembly 200 is tilted, and after the cover housing 400 is tilted to the second position, the cover housing 400 can always cover the gap region 1000a.

The cover housing 400 may only block a portion of the gap region 1000a. For example, the cover housing 400 may be in a shape of an elongated strip, and the elongated cover housing 400 may cover a top portion or a section of a top portion of the gap region 1000a. Alternatively, the cover housing 400 may include an upper covering portion and a side covering portion which extends downward from at least one of two opposite ends of the upper covering portion. In other words, the upper covering portion may have one side covering portion provided at only one end, or may have side covering portions provided at both opposite ends. The upper covering portion may cover the top portion or a section of the top portion of the gap region 1000a, and the side covering portion may block the side portion or a section of the side portion of the gap region 1000a. The cover housing 400, which only covers a portion of the gap region 1000a, mainly plays a role of preventing dust.

The cover housing 400 may also cover four sides of the gap region 1000a, which is equivalent to the cover housing 400 being in a ring shape which surrounds the gap region 1000a.

At the second position, the cover housing 400 may surround, and close or seal the gap region 1000a.

Exemplarily, the cover housing 400 may surround a periphery side of the gap region 1000a to form an enclosed space between the device body 100 and the screen assembly 200. Enclosed means that a space between the device body 100 and the screen assembly 200 is almost completely covered by the cover housing 400. However, the cover housing 400 may be provided with a structure, such as a ventilation hole, that enables the enclosed space to communicate with outside world. A gap that enables the enclosed space to communicate with the outside world may also be formed between the cover housing 400 and the device body 100, or between the cover housing 400 and the screen assembly 200. This type of cover housing 400 is mainly used to prevent dust.

Exemplarily, the cover housing 400 may also surround and seal the periphery side of the gap region 1000a to form a sealed space between the device body 100 and the screen assembly 200. Seal means that a gap between the device body 100 and the screen assembly 200 is completely covered by the cover housing 400.

The cover housing 400 has no structure, such as a ventilation hole, that enables the enclosed space to communicate with outside world; and no gap, that enables the enclosed space to communicate with outside world, is formed between the cover housing 400 and the device body 100 or between the cover housing 400 and the screen assembly 200. This type of cover housing 400 can not only prevent dust but also prevent water.

More preferably, a region which is covered by the cover housing 400 may be at least 50% of the gap region 1000a.

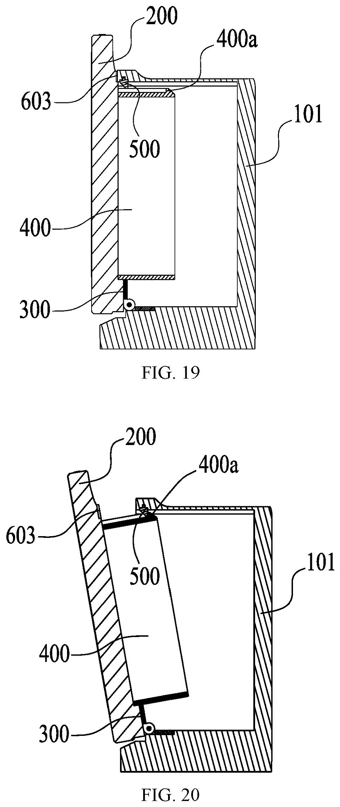

In one embodiment, referring to FIGS. 19, 20, 34 , 35 , 36 and 37, one end of the cover housing 400 is fixed to the mounting housing 201, and fixing described in this disclosure may be a fixed connection or an integral molding. At the first position, the cover housing 400 is accommodated inside the device housing 101. At the second position, at least a portion of the cover housing 400 extends out of the device housing 101 and covers the gap region 1000a. That is, at the first position, the cover housing 400 is accommodated inside the device housing 101, and while the screen assembly 200 is tilted, the cover housing 400 moves together with the screen assembly 200, so as to enable at least a portion of the cover housing 400 to extend out, so as to cover the gap region 1000a.

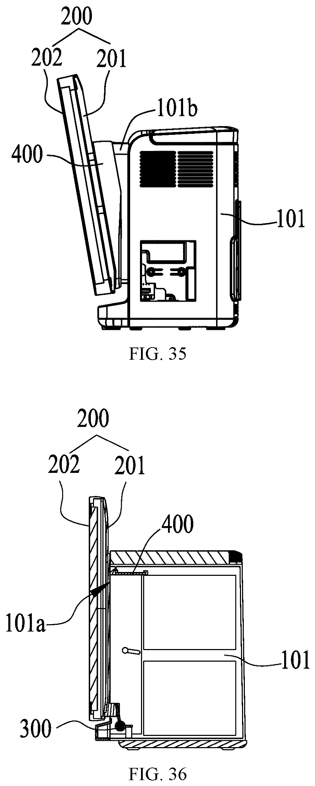

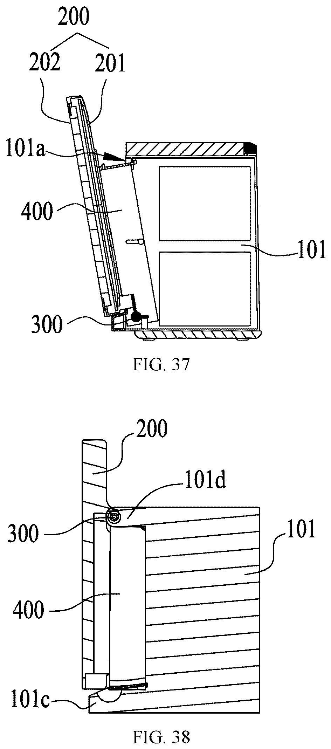

A front side of the device housing 101 may be provided with a first opening 101a as shown in FIGS. 36 and 37, or may be provided with a first groove. At the first position, the cover housing 400 extends into the device housing 101 from the first opening 101a or the first groove, so as to be accommodated inside the device housing 101.

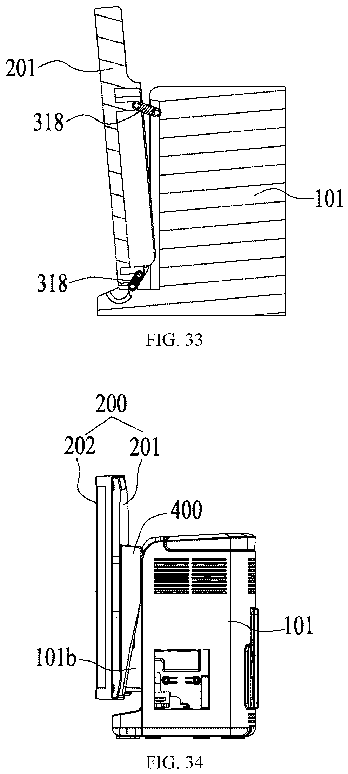

A structure for accommodating the cover housing 400 may also be provided at the device housing 101. For example, referring to FIGS. 34 and 35, a first sleeving portion 101b may be provided at a front side of the device housing 101. The cover housing 400 and the first sleeving portion 101b are movably sleeved with each other. At the first position, the cover housing 400 and the first sleeving portion 101b move close to each other, so as to enable at least a portion of the cover housing 400 to be accommodated inside the device housing 101. At the second position, the cover housing 400 and the first sleeving portion 101b move away from each other, so as to enable at least a portion of the cover housing 400 extends out of the device housing 101 and covers the gap region 1000a.

Specifically, a partial region of a front side of the device housing 101 may protrude forward to form a first sleeving portion 101b, or a front side of the device housing 101 may not protrude forward. For example, the first sleeving portion 101b may be arranged on the front side of the device housing 101 and be flush with a front surface of the device housing 101.

The cover housing 400 can be sleeved on an outer side of the first sleeving portion 101b, and can also be sleeved on an inner side of the first sleeving portion 101b. Wherein, for the cover housing 400 which is sleeved on an outer side of the first sleeving portion 101b; at the first position, the cover housing 400 is equivalent to being accommodated on the outer side of the device housing 101. That is to say, accommodation described in this disclosure refers to placing the cover housing 400 on the outer side or the inner side of the device housing 101 in a way that the gap region 1000a is reduced.

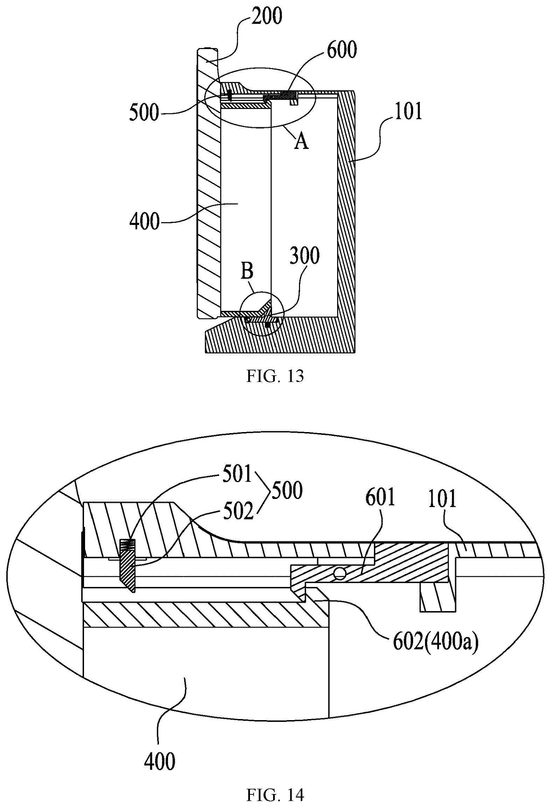

In one embodiment, please refer to FIGS. 13, 14, 16 and 17, the cover housing 400 can be provided with a sixth position-limiting portion 400a, and at least a portion of the cover housing 400, which portion is provided with the sixth position-limiting portion 400a, extends into the device housing 101. The monitoring device 1000 includes a position-limiting structure 500, which is arranged on an inner wall of the device housing 101, wherein the position-limiting structure 500 includes a first elastic member 501 and a first position-limiting member 502. The first elastic member 501 is configured to retract the first position-limiting member 502 to avoid the cover housing 400 during mounting the cover housing 400, so as to enable the sixth position-limiting portion 400a to move towards a rear side of the position-limiting structure 500. When the screen assembly 200 is tilted downward from the first position to the second position, the first position-limiting member 502 abuts against the sixth position-limiting portion 400a.

Specifically, please refer to FIG. 14. For the device housing 101 with a position-limiting structure 500, a front-back direction of the position-limiting structure 500 is perpendicular to an extension-retraction direction of the first elastic member 501. After the cover housing 400 is assembled, the sixth position-limiting portion 400a is always located at the rear side of the position-limiting structure 500 (i.e., a position of the sixth position-limiting portion 400a in FIG. 14). Therefore, while assembling the cover housing 400, when the cover housing 400 moves to a position in contact with the first position-limiting member 502, the cover housing 400 applies a force to the first position-limiting member 502 to retract the first elastic member 501, and the first position-limiting member 502 is retracted to avoid the cover housing 400, so as to enable a portion of the cover housing 400, at which portion the sixth position-limiting portion 400a is provided, to move from a front side of the position-limiting structure 500 to a rear side of the position-limiting structure 500. After a portion of the cover housing 400, at which portion the sixth position-limiting portion 400a is provided, has moved to the rear side of the position-limiting structure 500, the first elastic member 501 is stretched to drive the first position-limiting member 502 to extend, so as enable the first position-limiting member 502 to move to the front side of the sixth position-limiting portion 400a.

When the screen assembly 200 is tilted downward from the first position to the second position, the first position-limiting member 502 can implement a position limitation on the screen assembly 200 by abutting against the sixth position-limiting portion 400a, so as to enable the screen assembly 200 to stay at the second position.

It should be noted that a structural form and an arrangement position of the position-limiting structure 500 are not limited to the one described in the above embodiment. The monitoring device 1000 can also be provided with a position-limiting structure 500 of other structural forms, and the arrangement position of the position-limiting structure 500 can also be changed, as long as the screen assembly 200 can stay at the second position.

In one embodiment, one end of the cover housing 400 may be fixed to the device housing 101, and at least a portion of the cover housing 400 is accommodated inside the mounting housing 201 at the first position. At the second position, at least a portion of the cover housing 400 extends out of the mounting housing 201 and covers the gap region 1000a. That is to say, the cover housing 400 can also be accommodated inside the mounting housing 201. While the screen assembly 200 is tilted, the cover housing 400 remains stationary, which is equivalent to that the screen assembly 200 is tilted relative to the cover housing 400. Thus, at least a portion of the cover housing 400 can also extend out, so as to cover the gap region 1000a.

A second opening or a second groove may be provided on a rear side of the mounting housing 201. At the first position, the cover housing 400 extends into the mounting housing 201 from the second opening or the second groove, and is thus accommodated inside the mounting housing 201.

A structure for accommodating the cover housing 400 may also be provided on the mounting housing 201. For example, a second sleeving portion may be provided on a rear side of the mounting housing 201. The cover housing 400 and the second sleeving portion are movably sleeved with each other. At the first position, the cover housing 400 and the second sleeving portion move close to each other, so as to enable at least a portion of the cover housing 400 to be accommodated inside the device housing 101. At the second position, the cover housing 400 and the first sleeving portion 101b move away from each other, so as to enable at least a portion of the cover housing 400 to extend out of the mounting housing 201 and cover the gap region 1000a.

Specifically, a partial region of a rear side of the mounting housing 201 may protrude backward to form a second sleeving portion, or a rear side of the mounting housing 201 may not protrude backward. For example, the second sleeving portion may be arranged on the rear side of the mounting housing 201 and be flush with a rear surface of the mounting housing 201.

The cover housing 400 can be sleeved on an outer side of the second sleeving portion, or can be sleeved on an inner side of the second sleeving portion. For the cover housing 400 which is sleeved on an outer side of the second sleeving portion; at the first position, the cover housing 400 is equivalent to being accommodated on the outer side of the mounting housing 201.

It should be noted that, no matter if one end of the cover housing 400 is fixed to the mounting housing 201 or to the device housing 101, the accommodation methods of the cover housing 400 are not limited to those described in the above embodiments. The cover housing 400 can also adopt other accommodation methods, which are not limited here.

In one embodiment, a first opening 101a may be provided on a front side of the device housing 101, or a first groove may be provided on a front side of the device housing 101, and a second sleeving portion is provided on a rear side of the mounting housing 201. One end of the cover housing 400 extends into the device housing 101 from the first opening 101a or the first groove, and the second sleeving portion is sleeved on the other end of the cover housing 400, wherein the second sleeving portion may be sleeved on an outer side of the cover housing 400 or on an inner side of the cover housing 400, thereby achieving a fixation of the cover housing 400 to the mounting housing 201 and the device housing 101 respectively.

In one embodiment, a second opening may be provided on a rear side of the mounting housing 201, or a second groove may be provided on a rear side of the mounting housing 201; and a front side of the device housing 101 is provided with a first sleeving portion 101b. One end of the cover housing 400 extends into the mounting housing 201 from the second opening or the second groove, and the first sleeving portion 101b is sleeved on the other end of the cover housing 400, wherein the first sleeving portion 101b may be sleeved on an outer side of the cover housing 400 or on an inner side of the cover housing 400, thereby also achieving a fixation of the cover housing 400 to the mounting housing 201 and the device housing 101 respectively.

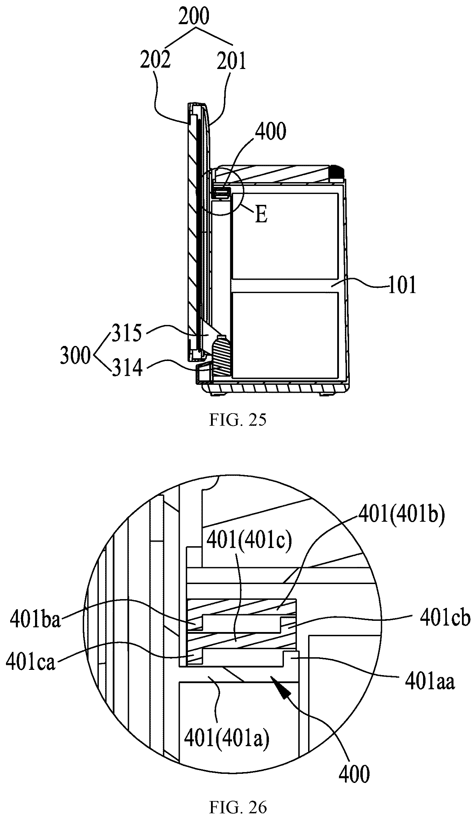

In one embodiment, please refer to FIGS. 25 to 29, one end of the cover housing 400 can be fixed to the mounting housing 201, and the other end of the cover housing 400 can be fixed to the device housing 101. The cover housing 400 has a plurality of sub-housings 401 that are movably connected in sequence, thereby covering the gap region 1000a and allowing the screen assembly 200 to be tilted from the first position to the second position relative to the device body 100.

That is to say, by a movement, the plurality of sub-housings 401 can not only cover the gap region 1000a, but also enable the screen assembly 200 to be tilted from the first position to the second position relative to the device body 100.

Exemplarily, referring to FIGS. 25 to 29, a plurality of sub-housings 401 can be movably connected in sequence in a sleeved manner, and the cover housing 400 has a state of moving close and a state of moving away; wherein in the state of moving close, the plurality of sub-housings 401 are respectively accommodated inside an adjacent sub-housing 401 in sequence; in the state of moving away, the plurality of sub-housings 401 move away from one another and adjacent sub-housings 401 maintains in connection with each other. The screen assembly 200 drives the cover housing 400 to switch between the state of moving close and the state of moving away by tilting relative to the device body 100.

Specifically, when the cover housing 400 shown in FIGS. 25 to 29 is in the state of moving close, the sub-housings 401 are stacked together, which is equivalent to the cover housing 400 being in a folded state. However, it should be noted that a folded state is only a type of a state of moving close. In other embodiments, the sub-housings 401 can move close to each other only in a manner that the gap region 1000a is reduced, but not stacked.

In addition, the screen assembly 200 shown in FIGS. 25 to 29 is tilted downward, so that the cover housing 400 is in a state of moving close at the first position, and is in a state of moving away at the second position. In other embodiments, the cover housing 400 may be in a state of moving away at the first position and in a state of moving close at the second position, for example, the screen assembly 200 is tilted upward. For another example, the screen assembly 200 is connected with the device body 100 adjacent to a top portion of the device body 100, and while the screen assembly 200 is tilted from the first position to the second position, a lower end of the screen assembly 200 is adjacent to the device body, and the cover housing 400 around the lower end of the screen assembly 200 gradually switches from a state of moving away to a state of moving close.

Further, referring to FIGS. 25 to 29, the plurality of sub-housings 401 include a first sub-housing 401a with one end fixed to the mounting housing 201, a second sub-housing 401b with one end fixed to the device housing 101, and a third sub-housing 401c located between the first sub-housing 401a and the second sub-housing 401b. The first sub-housing 401a has a first position-limiting portion 401aa, the second sub-housing 401b has a second position-limiting portion 401ba; the third sub-housing 401c has a third position-limiting portion 401ca on a side which is adjacent to the first sub-housing 401a, and a fourth position-limiting portion 401cb on a side which is adjacent to the second sub-housing 401b. One third sub-housing 401c exists in FIGS. 25 to 29. For the screen assembly 200 which is tilted downward, when the screen assembly 200 is tilted from the first position to the second position, the third position-limiting portion 401ca abuts against the first position-limiting portion 401aa, and the fourth position-limiting portion 401cb abuts against the second position-limiting portion 401ba, so as to implement a position limitation on the screen assembly 200.

In other embodiments, for the cover housing 400 in which at least two third sub-housings 401c exist, when the screen assembly 200 is tilted downward from the first position to the second position; one third position-limiting portion 401ca of the third sub-housing 401c, which is most adjacent to the first sub-housing 401a, may abut against the first position-limiting portion 401aa, and one fourth position-limiting portion 401cb of the third sub-housing 401c, which is most adjacent to the second sub-housing 401b, may abut against the second position-limiting portion 401ba, one third sub-housing of two adjacent third sub-housings 401c may abut against, through a third position-limiting portion 401ca of said third sub-housing, a fourth position-limiting portion 401cb of the other third sub-housing of said two adjacent third sub-housings.

In one embodiment, referring to FIGS. 38 to 41, the cover housing 400 may also be in rotatable connection with at least one of the mounting housing 201 and the device housing 101, that is, while the screen assembly 200 is tilted, the cover housing 400 may also have certain tilting.

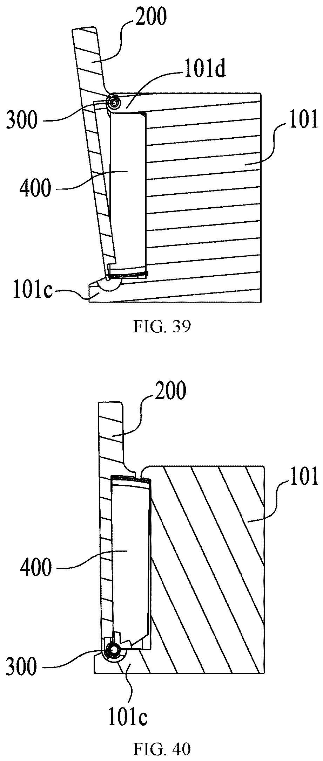

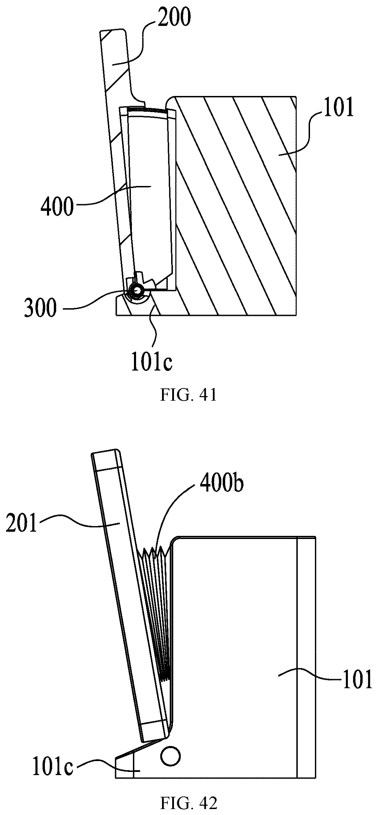

Please refer to FIGS. 40 and 41. The top surface of the cover housing 400 can be arranged on a curved surface, the screen assembly 200 can be slidably contacted with the curved surface, and the screen assembly 200 can slide along the curved surface while tilting.

In one embodiment, opposite sides of the cover housing 400 are respectively fixed to the mounting housing 201 and the device housing 101, and the screen assembly 200 is tilted relative to the device body 100, so as to drive the cover housing 400 to switch between folding and unfolding.

Different from the previous embodiment having multiple sub-housings 401 that are movably connected in sequence, the cover housing 400 described in this embodiment is an integral structure without multiple sub-housings 401. The cover housing 400 is made of flexible material(s) that can be folded and unfolded, such as one or more of canvas, leather, silicone, rubber, plastic and the likes.

For a screen assembly 200 that is tilted downward, the cover housing 400 is folded at the first position and unfolded at the second position. In other embodiments, the cover housing 400 may be unfolded at the first position and folded at the second position, for example, for a screen assembly 200 that is tilted upward. For another example, the screen assembly 200 is connected with the device body 100 adjacent to a top portion of the device body 100, and while the screen assembly 200 is tilted from the first position to the second position, a lower end of the screen assembly 200 is adjacent to the device body, and the cover housing 400 around the lower end of the screen assembly 200 gradually switches from a state of moving away to a state of moving close.

In one embodiment, the cover housing 400 may be provided with a foldable curtain and a support frame. When the screen assembly 200 is tilted from the first position to the second position, at least a portion of the screen assembly 200 gradually moves away from the device body 100, the cover housing 400 unfolds, and the support frame supports the foldable curtain.

The support frame can also be folded and unfolded together with the foldable curtain, but compared with the foldable curtain, the support frame is made of a relatively flexible material with greater hardness. When the cover housing 400 is unfolded, the support frame supports the foldable curtain to prevent the foldable curtain from collapsing.

Furthermore, the monitoring device 1000 can also be provided with a support member for an opening angle, which support member is in respective rotatable connection with the device housing 101 and the mounting housing 201. When the screen assembly 200 is tilted from the first position to the second position, the support member for an opening angle is supported between the device housing 101 and the mounting housing 201 to properly tighten the foldable curtain, thereby reducing creases of the foldable curtain and optimizing an appearance.

In some embodiments, the cover housing 400 may only have a foldable curtain but no support frame.

In one embodiment, please refer to FIG. 42, the cover housing 400 may also include a plurality of foldable portions 400b which are arranged in sequence, and a variable angle is formed between adjacent foldable portions 400b. While the screen assembly 200 is tilted; an angle between adjacent foldable portions 400b is gradually increased to unfold the cover housing 400, and an angle between adjacent foldable portions 400b is gradually decreased to fold the cover housing 400.

In one embodiment, at least one of the device housing 101, the mounting housing 201 and the connection assembly 300 may be provided with a mounting portion of the cover housing for mounting the cover housing 400. When the cover housing 400 is mounted on the mounting portion of the cover housing, the cover housing 400 covers a gap region 1000a, which is formed between the device body 100 and the screen assembly 200 due to tilting of the screen assembly 200 relative to the device body 100.

The mounting portion of the cover housing is a portion which is connected with the cover housing 400. According to requirements, the mounting portion of the cover housing can be arranged at one of the device housing 101, the mounting housing 201 and the connection assembly 300, which is equivalent to the cover housing 400 being connected with one of the device housing 101, the mounting housing 201 and the connection assembly 300. A mounting portion of the cover housing can also be respectively arranged at two of the device housing 101, the mounting housing 201 and the connection assembly 300, which is equivalent to the cover housing 400 being connected with two of the device housing 101, the mounting housing 201 and the connection assembly 300. A mounting portion of the cover housing can also be arranged at the device housing 101, the mounting housing 201 and the connection assembly 300, which is equivalent to the cover housing 400 being respectively connected with the device housing 101, the mounting housing 201 and the connection assembly 300.

The cover housing 400 can be in detachable connection with the mounting portion of the cover housing, that is, medical personnel or maintenance personnel can remove the cover housing 400 from the monitoring device 1000 as needed, or mount the cover housing 400 between the device housing 101 and the mounting housing 201. When the cover housing 400 is mounted between the device housing 101 and the mounting housing 201, the gap region 1000a can be covered.

A structural form of the mounting portion of the cover housing is not limited. For example, the mounting portion of the cover housing can be one of a snap-fit, a plug-in slot, and a fastening connection hole. That is, the cover housing 400 can be snapped, plugged, or fastened to the mounting portion of the cover housing by using fasteners such as screws.

In addition, for the cover housing 400 which is detachable, the cover housing 400 can be manufactured and sold independently, that is, the monitoring device 1000 purchased by a user can be without the cover housing 400, and the cover housing 400 is sold separately as an optional accessory.

Exemplarily, when the cover housing 400 is mounted at the mounting portion of the cover housing, while the screen assembly 200 is tilted downward from the first position to the second position, an upper edge of the screen assembly 200 gradually moves away from the device body 100, and a gap region 1000a, which is formed between the screen assembly 200 and the device body 100, gradually increases, and the cover housing 400 always covers the gap region 1000a.

In one embodiment, referring to FIG.S 13 to 20, the monitoring device 1000 can be provided with a position-restricting structure 600, which is configured to restrict the screen assembly 200 to a first position, a second position, or at least one intermediate position between the first position and the second position, so as to restrict the screen assembly 200 from rotating relative to the device body 100.

The intermediate position is another position between the first position and the second position, before tilting to the second position.

For example, assuming that the screen assembly 200 is at an angle of 0° at the first position, and is capable of being tilted for 15° from the first position to the second position, intermediate positions can be 5°, 8°, 10°, 12°, and so on.

The position-restricting structure 600 may be used only to restrict the screen assembly 200 to the first position, or may be used only to restrict the screen assembly 200 to the second position, or may be used only to restrict the screen assembly 200 to at least one intermediate position.

The position-restricting structure 600 can also be used to restrict the screen assembly 200 to multiple positions, that is, the position-restricting structure 600 can restrict the screen assembly 200 to at least two positions among the first position, the second position and at least one intermediate position.

That is to say, the position-restricting structure 600 can play a role in making the screen assembly 200 stay at a corresponding position.

It should be noted that, in some embodiments, the position-restricting structure 600 can also play a position-limiting role, that is, the position-restricting structure 600 has a similar function to the position-limiting structure 500. For example, when the position-restricting structure 600 is configured to restrict the screen assembly 200 to the second position, the position-restricting structure 600 actually also plays a position-limiting role at the same time.

Exemplarily, referring to FIGS. 13 to 20, the position-restricting structure 600 restricts the screen assembly 200 at the first position, that is, the position-restricting structure 600 can make the screen assembly 200 stay at the first position, and when the screen assembly 200 is released from the restriction of the position-restricting structure 600, the screen assembly 200 can be tilted to the second position.

A structure of the position-restricting structure 600 is not limited, as long as it can restrict the screen assembly 200 to the first position. For example, in one embodiment, please refer to FIGS. 13 to 18, the position-restricting structure 600 includes a first locking member 601 and a second locking member 602, wherein the first locking member 601 is movably arranged at one of the screen assembly 200 and the device body 100, and the second locking member 602 is arranged at the other of the screen assembly 200 and the device body 100, and the first locking member 601 is locked and unlocked with the second locking member 602 through a movement of the first locking member 601.

The first locking member 601 being movably arranged at one of the screen assembly 200 and the device body 100, means that the first locking member 601 can be arranged at the mounting housing 201 or the device housing 101. For the monitoring device 1000 with a cover housing 400, if the cover housing 400 is fixed to the mounting housing 201, the first locking member 601 can be arranged at the cover housing 400, so as to be indirectly arranged at the screen assembly 200. If the cover housing 400 is fixed to the device housing 101, the first locking member 601 can be arranged at the cover housing 400, so as to be indirectly arranged at the device body 100.

The second locking member 602 in FIGS. 13 to 18 is also a sixth position-limiting portion 400a at the cover housing 400, which portion fits with the position-limiting structure 500, which is equivalent to the second locking member 602 and the sixth position-limiting portion 400a being combined into one. In other embodiments, the second locking member 602 and the sixth position-limiting portion 400a can also be arranged separately.

Similarly, the second locking member 602 being arranged at the other of the screen assembly 200 and the device body 100, means that the second locking member 602 can be arranged at the mounting housing 201 or the device housing 101. For the monitoring device 1000 with a cover housing 400, if the cover housing 400 is fixed to the mounting housing 201, the second locking member 602 can be arranged at the cover housing 400, so as to be indirectly arranged at the screen assembly 200. If the cover housing 400 is fixed to the device housing 101, the second locking member 602 can be arranged at the cover housing 400, so as to be indirectly arranged at the device body 100.

When the first locking member 601 and the second locking member 602 are locked with each other, the screen assembly 200 is restricted to the first position. When the first locking member 601 and the second locking member 602 are unlocked with each other, the screen assembly 200 is released from the restriction of the position-restricting structure 600.

Exemplarily, referring to FIGS. 13 to 18, the first locking member 601 and the second locking member 602 can be locked with each other by abutting against each other, and the first locking member 601 is rotated relative to the second locking member 602 under an action of an external force, so as to be switched to an unlocking state, in which the first locking member 601 is separated from the second locking member 602. That is to say, in the locking state, medical staff can press to rotate the first locking member 601 relative to the second locking member 602, thereby achieving unlocking.

In another embodiment, please refer to FIGS. 19 and 20, the screen assembly 200 can also be released from the restriction of the position-restricting structure 600 under a push of an external force. That is to say, the position-restricting structure 600 does not restrict the screen assembly 200 to the first position by locking. The medical staff only needs to push the screen assembly 200 to release the screen assembly 200 from the restriction of the position-restricting structure 600.

Exemplarily, the position-restricting structure 600 includes a magnetic member 603, which is fixed to at least one of the screen assembly 200 and the device body 100, wherein the screen assembly 200 and the device body 100 are fitted with each other through magnetic attraction via the magnetic member 603.

The magnetic member 603 can be arranged at the mounting housing 201 and/or the device housing 101. For the monitoring device 1000 with a cover housing 400, if the cover housing 400 is fixed to the mounting housing 201, the magnetic member 603 can be fixed to the cover housing 400, so as to be indirectly fixed to the screen assembly 200. If the cover housing 400 is fixed to the device housing 101, the magnetic member 603 can be fixed to the cover housing 400, so as to be indirectly fixed to the device body 100.

Similarly, the mounting housing 201 or the device housing 101 may be fitted with the magnetic member 603 through magnetic attraction; or the cover housing 400, which is fixed to the mounting housing 201 or the device housing 101, may be fitted with the magnetic member 603 through magnetic attraction.

It is understandable that a portion of the mounting housing 201 or of the device housing 101, which portion is fitted with the magnetic member 603 through magnetic attraction, can be a structure that can be fitted with the magnetic member 603 through magnetic attraction, for example, the portion of the mounting housing 201 or of the device housing 101, which portion is fitted with the magnetic member 603 through magnetic attraction, is made of metal.

Exemplarily, the position-restricting structure 600 may also include a first magnetic member and a second magnetic member, which are fitted with each other through magnetic attraction, wherein the first magnetic member is fixed to the screen assembly 200, and the second magnetic member is fixed to the device body 100.

The first magnetic member can be fixed to the mounting housing 201. For the monitoring device 1000 with a cover housing 400, if the cover housing 400 is fixed to the mounting housing 201, the first magnetic member can also be fixed to the cover housing 400, so as to be indirectly fixed to the screen assembly 200. The second magnetic member can be fixed to the device housing 101. For the monitoring device 1000 with a cover housing 400, if the cover housing 400 is fixed to the device housing 101, the second magnetic member can also be fixed to the cover housing 400, so as to be indirectly fixed to the device body 100.

That is to say, the screen assembly 200 can be restricted to the first position by magnetic attraction. The medical staff only needs to push the screen assembly 200, so as to enable the screen assembly 200 to overcome a magnetic attraction force to release the restriction of the position-restricting structure 600.

For the monitoring device 1000 with a connection assembly 300, an arrangement position of the connection assembly 300 is not limited, as long as the connection assembly 300 can tilt the screen assembly 200.

Exemplarily, referring to FIGS. 1 to 4 and FIGS. 43 to 46, for the screen assembly 200 which is tilted downward, while the screen assembly 200 is tilted downward from the first position to the second position, a distance between an upper edge of the screen assembly 200 and the device body 100 gradually increases, and a distance between a lower edge of the screen assembly 200 and the device body 100 gradually decreases, and wherein, when the screen assembly 200 is tilted to the second position, the screen assembly 200 is in contact with the device housing 101 or a gap is formed between the screen assembly 200 and the device housing 101. That is to say, at the second position, the screen assembly 200 can be in contact with the device housing 101 or a gap can be formed between the screen assembly 200 and the device housing 101, which is equivalent to that an arrangement position of the connection assembly is acceptable as long as there is space left between the screen assembly 200 and the device housing 101 for the screen assembly 200 to tilt.

It should be noted that the screen assembly 200 is in contact with the device housing 101 on the premise that at least component(s) of the monitoring device 1000 is(are) not damaged due to the contact between the screen assembly 200 and the device housing 101.

Exemplarily, the screen assembly 200 may be in contact with the device housing 101 or a gap may be formed between the screen assembly 200 and the device housing 101, by having the connection assembly 300 be mounted on a front side of the device housing 101, in such a manner that at least a portion of the connection assembly 300 protrudes outward from the front side of the device housing 101 for a first distance, so as to enable a lower side of the screen assembly 200 to have a sufficient movement space, while the screen assembly 200 is tilted downward from the first position to the second position. For example, referring to FIGS. 1 to 4 and 43 to 46, a partial region of the device housing 101 may protrude from the front side of the device housing 101 to form a connection portion 101d above the ground, and the connection assembly 300 may be arranged at the connection portion 101d.

Further, referring to FIGS. 45 and 46, a distance between a horizontal center line of the connection portion 101d and a horizontal center line of the monitoring device 1000 may be respectively less than a distance between the horizontal center line of the connection portion 101d and a top end surface of the monitoring device 1000, and a distance between the horizontal center line of the connection portion 101d and a bottom end surface of the monitoring device 1000; a lower side of the connection portion 101d has a movement space for the screen assembly 200 to be tilted to the second position. That is to say, the connection portion 101d is roughly located at a middle position of the monitoring device 1000, and the connection portion 101d is neither adjacent to the top surface nor the bottom surface of the monitoring device 1000. Therefore, before and after the tilting of the screen assembly 200, it can be ensured that a center of gravity of the monitoring device 1000 will hardly shift, so as to improve an overall stability of the monitoring device 1000.

Exemplarily, the screen assembly 200 may be in contact with the device housing 101 or a gap may be formed between the screen assembly 200 and the device housing 101, in such a manner that the device housing 101 has a shape which enables a lower side of the screen assembly 200 to have a sufficient movement space, while the screen assembly 200 is tilted downward from the first position to the second position.

Illustratively, on a projection plane which is perpendicular to a tilting axis of the screen assembly 200, at least a portion of a contour line of the front side of the device housing 101 may be inclined from top to bottom toward a rear side of the device housing 101, that is, along a front-to-rear direction of the device housing 101, a size of the lower side of the device housing 101 may be less than a size of the upper side of the device housing 101, so as to enable the lower side of the device housing 101 to have a sufficient movement space.

Exemplarily, at least a portion of the front side of the device housing 101 may also be recessed toward the rear side of the device housing 101 to form a space for the lower side of the screen assembly 200 to move.

Exemplarily, for the device housing 101 provided with a convex portion 101c, the connection assembly 300 may also be provided at the convex portion 101c.

In addition, in addition to the connection portion 101d and the convex portion 101c, the connection assembly 300 can also be arranged at other positions. For example, the connection assembly 300 can be arranged on a surface of a front side of the device housing 101, or at least a portion of the connection assembly 300 can also be arranged inside the device housing 101.

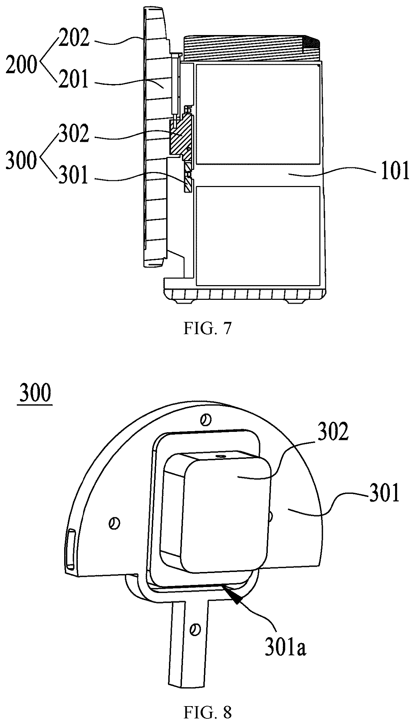

The connection assembly 300 may also have various structural forms. For example, in one embodiment, please refer to FIGS. 7 to 9. The connection assembly 300 includes a fixing block 301 fixed to the device housing 101 and a rotating block 302 fixed to the mounting housing 201. The rotating block 302 is in rotatable connection with the fixing block 301. That is, the rotating block 302 drives the screen assembly 200 to tilt by rotating relative to the fixing block 301.

Please refer to FIG. 7 to FIG. 9. For the screen assembly 200 which is tilted downward, when the screen assembly 200 is tilted downward to the second position, the rotating block 302 abuts against the device housing 101 to implement a position-limitation on the screen assembly 200.

Exemplarily, referring to FIGS. 7 to 9, the fixing block 301 has a mounting opening 301a, the rotating block 302 rotatably penetrates through and is arranged inside the mounting opening 301a, wherein the rotating block 302 and a region of the device housing 101 which region is located at the mounting opening 301a, are arranged with a gap therebetween, which gap forms a space for the rotating block 302 to rotate therein.

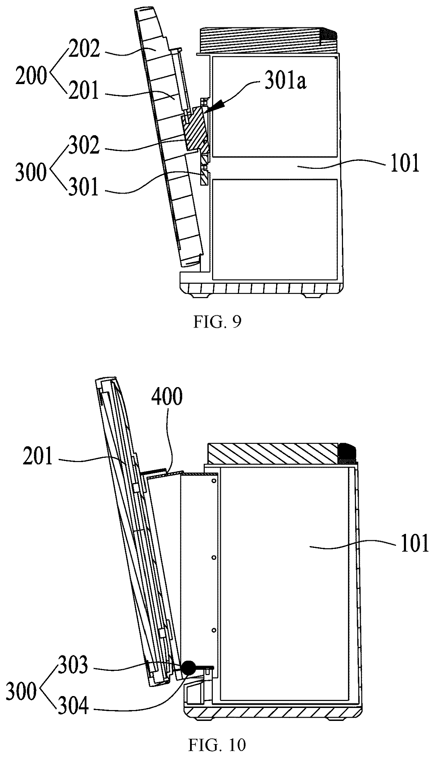

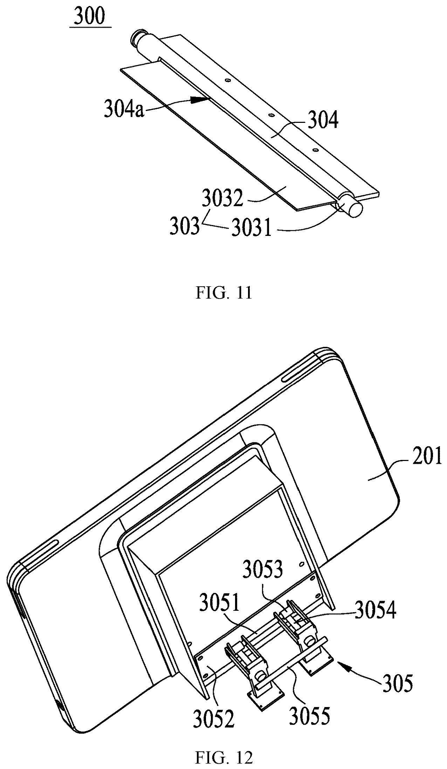

In one embodiment, please refer to FIGS. 10 and 11, the connection assembly 300 includes a damping shaft 303 and a connection column 304 with a shaft hole. In FIGS. 10 and 1, the damping shaft 303 is fixed to the mounting housing 201, and the connection column 304 is fixed to the device housing 101. In some embodiments, the connection column 304 may also be fixed to the mounting housing 201, and the damping shaft 303 may be fixed to the device housing 101. The damping shaft 303 rotatably penetrates through and is arranged inside the shaft hole.

The damping shaft 303 has a certain damping, which enables the screen assembly 200 to stay at the first position, the second position, and any position between the first position and the second position, which is equivalent to the screen assembly 200 being able to achieve infinite adjustment of angle.

Please refer to FIGS. 10 and 11. A position-limiting groove 304a, which extends along an axial direction and is connected with the shaft hole, can be arranged on a side wall of the connection column 304. The damping shaft 303 includes a shaft body 3031, and a position-limiting plate 3032, which is arranged at a side wall of the shaft body 3031. The shaft body 3031 rotatably penetrates through and is arranged inside the shaft hole, and the position-limiting plate 3032 penetrates through the position-limiting groove 304a to extend out of the connection column 304. When the screen assembly 200 is tilted to the first position or the second position, the position-limiting plate 3032 abuts against the connection column 304. That is to say, the position-limiting plate 3032 fits with the position-limiting groove 304a. When the screen assembly 200 is tilted to the first position, the position-limiting plate 3032 abuts against the connection column 304. When the screen assembly 200 is tilted to the second position, the position-limiting plate 3032 also abuts against the connection column 304. Thus, the screen assembly 200 can only be tilted between the first position and the second position, which is equivalent to the position-limiting plate 3032 fitting with the position-limiting groove 304a to control a maximum tilting angle of the screen assembly 200.

In one embodiment, please refer to FIG. 12, the connection assembly 300 includes a driving motor (not shown) and a transmission mechanism 305. The driving motor drives the transmission mechanism 305 to move, so as to enable the transmission mechanism 305 to drive the screen assembly 200 to tilt. In other words, the screen assembly 200 can be driven to tilt by electrical control.

In one embodiment, please refer to FIG. 12, the transmission mechanism 305 includes a connection shaft 3051, an adapter plate 3052, a worm wheel 3053 and a worm rod 3054, the worm wheel 3053 penetrates through and is arranged at the connection shaft 3051, the worm rod 3054 is meshed with the worm wheel 3053, the adapter plate 3052 is respectively connected with the connection shaft 3051 and the mounting housing 201, and the driving motor is in drive connection with the worm rod 3054. That is to say, a fitting between the worm rod 3054 and the worm wheel 3053 can be used to drive the screen assembly 200 to tilt.

The transmission mechanism 305 of FIG. 12 is provided with two sets of worm rod 3054 and worm wheel 3053. In some embodiments, the transmission mechanism 305 may also be provided with one set of worm rod 3054 and worm wheel 3053, or more than two sets of worm rod 3054 and worm wheel 3053.

Further, referring to FIG. 12, the transmission mechanism 305 includes a driving rod 3055 meshed with the worm rod 3054, and the driving motor is connected with the driving rod 3055 to drive the driving rod 3055 to rotate, so as to further drive the worm rod 3054 to rotate through the driving rod 3055. By providing the driving rod 3055, it is convenient to arrange the driving motor in a direction perpendicular to an extension direction of the driving rod 3055. In some embodiments, the driving rod 3055 may not be provided, but the driving motor may be directly connected with the worm rod 3054.

It should be noted that the transmission mechanism 305 is not limited to the worm rod 3054 and the worm wheel 3053. In some embodiments, the transmission mechanism 305 may also be a gear set, or a combination of a gear set, a worm rod 3054 and a worm wheel 3053.

In addition, it can be understood that, in addition to the connection assembly 300 explicitly described as using a driving motor, the connection assembly 300 in other embodiments of this disclosure can tilt the screen assembly 200 manually or in combination with an electric method.

In one embodiment, referring to FIGS. 13 to 18, for the monitoring device 1000 having a position-restricting structure 600, the connection assembly 300 can fit with the position-restricting structure 600. For example, when the screen assembly 200 is released from the restriction of the position-restricting structure 600, the connection assembly 300 pushes the screen assembly 200 to tilt to the second position. That is to say, when the screen assembly 200 is released from the restriction of the position-restricting structure 600, the connection assembly 300 can automatically push the screen assembly 200 to tilt to the second position.

It should be noted that the pushing described in this embodiment refers to that the screen assembly 200 can be tilted under a push force of the connection assembly 300, and rather than the connection assembly 300 needs to contact with the screen assembly 200. For example, the cover housing 400 in FIGS. 13 to 18 is fixed to the screen assembly 200, and the connection assembly 300 is actually in contact with the cover housing 400, so as to tilt the screen assembly 200 by directly pushing the cover housing 400. In other embodiments, the connection assembly 300 may also be in contact with the screen assembly 200, so as to tilt the screen assembly 200 by directly pushing the screen assembly 200.

In one embodiment, referring to FIGS. 13 to 18, the connection assembly 300 includes a pushing member 306 and a second elastic member 307. When the screen assembly 200 is released from the restriction of the position-restricting structure 600, the pushing member 306 pushes the screen assembly 200 to tilt to the second position under an elastic force of the second elastic member 307. That is to say, the elastic force of the second elastic member 307 can be used to push the screen 202 group to tilt to the second position.

Exemplarily, referring to FIGS. 13 to 18, the pushing member 306 includes a rod portion 3061 and a pushing portion 3062. The rod portion 3061 is rotatably arranged at the device housing 101, the pushing portion 3062 is arranged on one side of the rod portion 3061 and faces the screen assembly 200, the second elastic member 307 is arranged at a side of the rod portion 3061 which side is away from the pushing portion 3062. The pushing portion 306 rotates under the elastic force of the second elastic member 307 to push the screen assembly 200 to tilt.

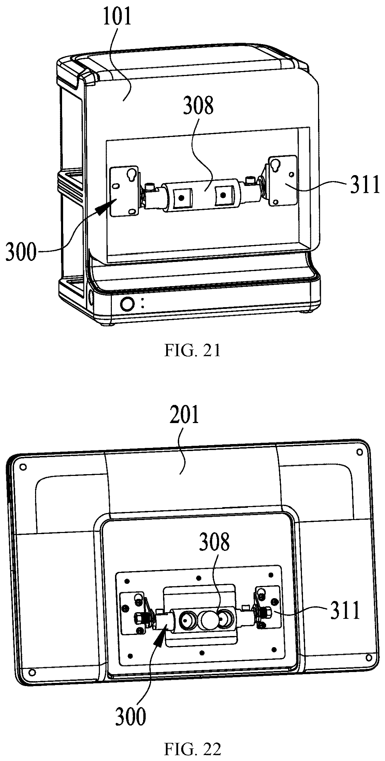

In one embodiment, please refer to FIGS. 21 to 24, the connection assembly 300 includes a connection frame 308, a rotation shaft 309, a damping plate 310, a fixing member 311 and an adjustment nut 312; wherein the rotation shaft 309 is in fixing connection with the connection frame 308; the fixing member 311 and the damping plate 310 penetrate through and are arranged at the rotation shaft 309; the fixing member 311 can rotate relative to the rotation shaft 309; the adjustment nut 312 is located on a side of the damping plate 310 which side is away from the fixing member 311, and is in threaded connection with the rotation shaft 309. In FIGS. 21 to 24, the connection frame 308 is in fixing connection with the device housing 101, and the fixing member 311 is in fixing connection with the mounting housing 201. In some embodiments, the connection frame 308 may be in fixing connection with the mounting housing 201, and the fixing member 311 may be in fixing connection with the device housing 101.

It should be noted that the connection frame 308 is in fixing connection with the device housing 101, so that a weight of the connection assembly 300 can be concentrated on the device body 100. For the screen assembly 200 which is downward tilted, a risk, that the monitoring device 1000 loses its center of gravity due to downward tilting of the screen assembly 200, can be effectively reduced.

The damping sheet 310 is used to provide damping for tilting of the screen assembly 200, so as to enable the screen assembly 200 to stay at a first position, a second position, and any position between the first position and the second position, which is equivalent to the screen assembly 200 being able to achieve infinite adjustment of angle.

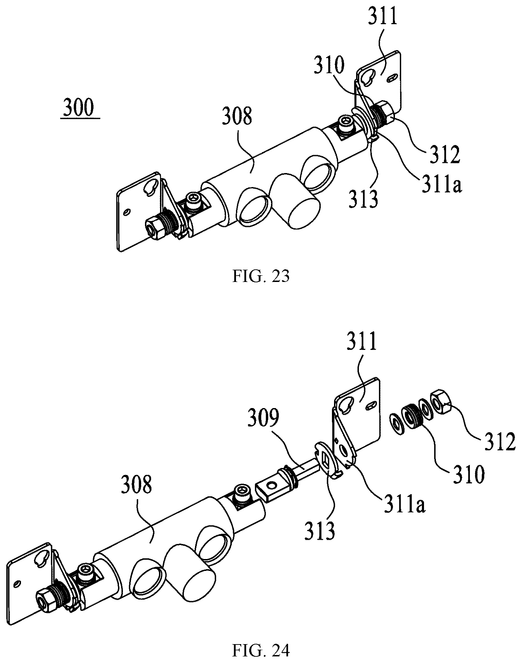

Please refer to FIGS. 23 and 24. The fixing member 311 can also be provided with a fifth position-limiting portion 311a. The connection assembly 300 also includes a second position-limiting member 313, which is fixed to the rotation shaft 309 and located on a side of the fixing member 311. When the fixing member 311 rotates to a first position or a second position, the fifth position-limiting portion 311a abuts against the second position-limiting member 313.

In one embodiment, referring to FIGS. 25, 28, 36 and 37, the connection assembly 300 may also be a hinge, which is in respective fixing connection with the screen assembly 200 and the device body 100.

The hinge can be fixed to the mounting housing 201. For the monitoring device 1000 with a cover housing 400, if the cover housing 400 is fixed to the mounting housing 201, the hinge can also be fixed to the cover housing 400, so as to be indirectly fixed to the screen assembly 200. Similarly, the hinge can be fixed to the device housing 101. For example, the hinge can be mounted at a front end or a rear end of the convex portion 101c, or at a mounting portion. For the monitoring device 1000 with the cover housing 400, if the cover housing 400 is fixed to the device housing 101, the hinge can also be fixed to the cover housing 400, so as to be indirectly fixed to the device body 100.

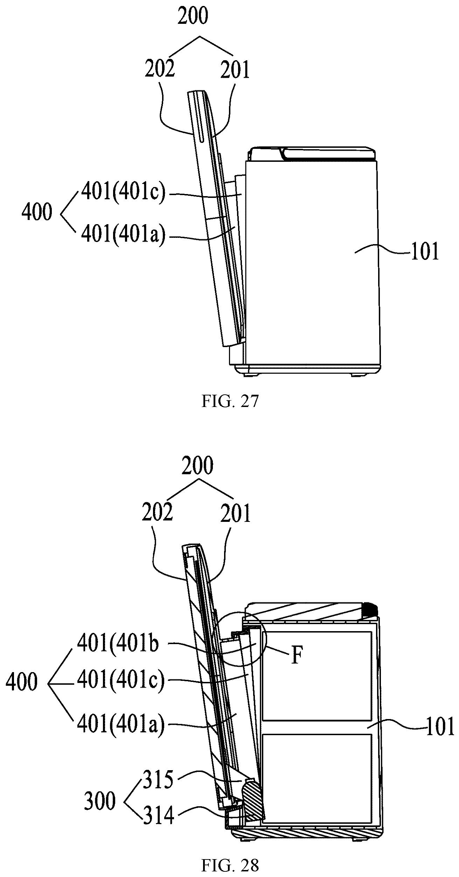

Exemplarily, referring to FIGS. 25 and 28, the hinge may include a fixing arm 314 and a connection arm 315, the fixing arm 314 is fixed to the device body 100, one end of the connection arm 315 is in rotatable connection with the fixing arm 314, and the other end of the connection arm 315 is in fixing connection with the mounting housing 201.

Exemplarily, referring to FIG. 36 and FIG. 37, the hinge may also be a butt hinge.

In one embodiment, for a monitoring device 1000 with a cover housing 400, the connection assembly 300 can also be in rotatable connection with the cover housing 400. For example, the cover housing 400 can form a rectangle ring with a certain thickness. The connection assembly 300 is in rotatable connection with the cover housing 400 through a mounting axis, and the mounting housing 201 of the screen assembly 200 is fixed to the connection assembly 300.

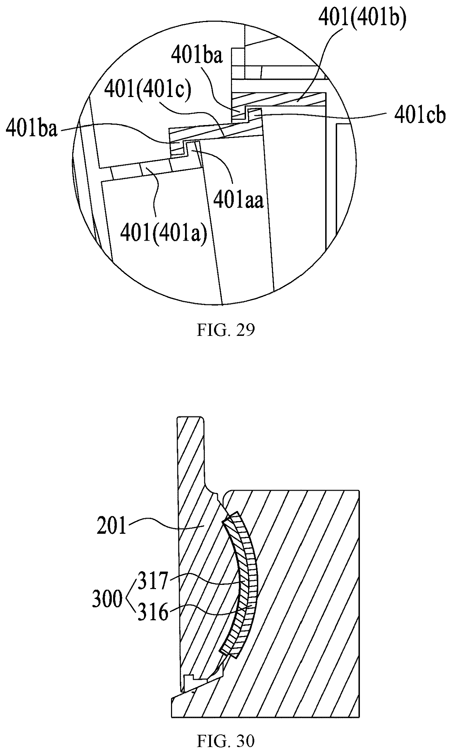

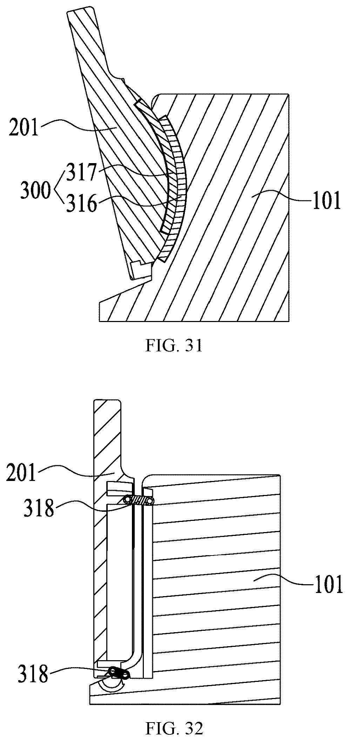

In one embodiment, referring to FIGS. 30 and 31, the connection assembly 300 includes a first slidable portion 316 and a second slidable portion 317 which are slidably fitted with each other, wherein the first slidable portion 316 is arranged at the device housing 101, and the second slidable portion 317 is arranged at the mounting housing 201, so as to enable the screen assembly 200 to be tilted relative to the device body 100, so as to adjust an angle between the screen assembly 200 and a horizontal plane.

That is to say, the screen assembly 200 is tilted by sliding relative to the device body 100, and this tilting method is actually an irregular circular movement of a movement trajectory of a center of mass of the screen assembly 200.

Exemplarily, referring to FIG. 30 and FIG. 31, the first slidable portion 316 may be arc-shaped, and the second slidable portion 317 is slidably fitted with the first slidable portion 316, so as to enable the screen assembly 200 to be tilted relative to the device body 100 by sliding.

The first slidable portion 316 in FIG. 30 and FIG. 31 is an arc-shaped portion that is recessed toward a rear side of the device housing 101. In some embodiments, the first slidable portion 316 may also be an arc-shaped portion which protrudes toward a front side of the device housing 101.

Structural forms of the first slidable portion 316 and the second slidable portion 317 are not limited, as long as they are slidably fitted with each other. For example, in one embodiment, the first slidable portion 316 may be a slide rail, and the second slidable portion 317 may be a slidable member that slides along the slide rail, such as a slider, a pulley, and the likes.

In another embodiment, the first slidable portion 316 may also be a slide groove, and the second slidable portion 317 is a slidable member which extends into the slide groove, such as a slider, a pulley, etc.

In one embodiment, referring to FIG. 30 and FIG. 31, a shape of a rear side of the mounting housing 201 matches a shape of a front side of the device housing 101, and there is a space between the mounting housing 201 and the device housing 101 to allow the screen assembly 200 to tilt.

In one embodiment, referring to FIG. 32 and FIG. 33, the connection assembly 300 includes a plurality of connection rods 318, and each connection rod 318 is in respective rotatable connection with the mounting housing 201 and the device housing 101. That is, the tilting of the screen assembly 200 can be achieved by utilizing a connection rod structure.

The tilting achieved by the connection rod structure is that the screen assembly 200 performs a circular movement around two or more axes.

A number of connection rods 318 is not limited as long as the tilting of the screen assembly 200 can be achieved. Exemplarily, referring to FIGS. 32 and 33, a number of connection rods 318 can be four, wherein two connection rods 318 are respectively arranged on opposite sides of the device housing 101 along a horizontal direction, and the two connection rods 318 located on a same side of the device housing 101 are respectively arranged on a top side and a bottom side of the device housing 101.

In the description of this disclosure, reference terms such as "in one embodiment", "in some embodiments", "in other embodiments", or " exemplarily " mean that specific features, structures, materials, or characteristics described in conjunction with the embodiment or example are included in at least one embodiment or example of the embodiments of this disclosure. In this disclosure, the exemplary expressions of the above terms do not necessarily refer to a same embodiment or example. Furthermore, the specific features, structures, materials, or characteristics described may be combined in any suitable manner in any one or more embodiments or examples. Furthermore, those skilled in the art may combine different embodiments or examples described in this disclosure and features of different embodiments or examples without mutual contradiction.

The above are only preferred embodiments of this disclosure and are not used to limit the protection scope of this disclosure. For those skilled in the art, this disclosure may have various modifications and changes. Any modifications, equivalent substitutions, improvements, etc., made within the spirit and principles of this disclosure are included in the protection scope of this disclosure.

## Claims

1. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a mounting housing and a screen which is arranged at the mounting housing;
a connection assembly, wherein the screen assembly is in movable connection with the device body through the connection assembly, so as to enable the screen assembly to be tilted from a first position to a second position relative to the device body; wherein when the screen assembly is tilted from the first position to the second position, at least a portion of the screen assembly gradually moves away from or close to the device body.

2. The monitoring device according to claim 1, **characterized in that**:
the connection assembly is a cover housing; wherein the cover housing is configured to cover a gap region, which is formed between the device body and the screen assembly due to tilting of the screen assembly relative to the device body; or
the monitoring device further comprises a cover housing; wherein the cover housing is arranged between the device housing and the mounting housing, or is arranged at at least one of the device housing, the mounting housing and the connection assembly, so as to cover a gap region, which is formed between the device body and the screen assembly due to tilting of the screen assembly relative to the device body.

3. The monitoring device according to claim 1, **characterized in that**, the connection assembly is in respective connection with the screen assembly and the device body; or the connection assembly is a portion of the screen assembly or a portion of the device body.

4. The monitoring device according to claim 1, **characterized in that**, the first position is a position at which the screen assembly is in a vertical state, and when the screen assembly is at the first position, an angle between the screen assembly and a top surface of the device body is 80-100 degrees.

5. The monitoring device according to claim 2, **characterized in that**, the screen assembly is tilted downward from the first position to the second position, and while the screen assembly is tilted, an upper edge of the screen assembly gradually moves away from the device body, and the gap region, which is formed between the screen assembly and the device body, gradually increases.

6. The monitoring device according to any one of claims 2-5, **characterized in that**, at the second position, the cover housing surrounds, and encloses or seals the gap region.

7. The monitoring device according to any one of claims 2-5, **characterized in that**, one end of the cover housing is fixed to the mounting housing; at the first position, the cover housing is accommodated inside the device housing, and, at the second position, at least a portion of the cover housing extends out of the device housing and covers the gap region.

8. The monitoring device according to claim 7, **characterized in that**:
a front side of the device housing is provided with a first opening or a first groove; at the first position, the cover housing extends into the device housing from the first opening or the first groove, so as to be accommodated inside the device housing; or
a front side of the device housing is provided with a first sleeving portion, and the cover housing is movably sleeved with the first sleeving portion; at the first position, the cover housing and the first sleeving portion move close to each other, so as to enable at least a portion of the cover housing to be accommodated inside the device housing; at the second position, the cover housing and the first sleeving portion move away from each other, so as to enable at least a portion of the cover housing to extend out of the device housing and cover the gap region.

9. The monitoring device according to claim 7, **characterized in that**, the screen assembly is tilted downward from the first position to the second position, the cover housing is provided with a sixth position-limiting portion; wherein at least a portion of the cover housing, which portion is provided with the sixth position-limiting portion, extends into the device housing;
the monitoring device further comprises a position-limiting structure which is arranged on an inner wall of the device housing; wherein the position-limiting structure comprises a first elastic member and a first position-limiting member, wherein the first elastic member is configured to enable the first position-limiting member to avoid the cover housing by retracting while the cover housing is mounted, so as to enable the sixth position-limiting portion to move to a rear side of the position-limiting structure; when the screen assembly is tilted downward from the first position to the second position, the first position-limiting member abuts against the sixth position-limiting portion.

10. The monitoring device according to any one of claims 2-5, **characterized in that**, one end of the cover housing is fixed to the device housing; at the first position, at least a portion of the cover housing is accommodated inside the mounting housing, and, at the second position, at least a portion of the cover housing extends out of the mounting housing and covers the gap region.

11. The monitoring device according to claim 10, **characterized in that**:
a rear side of the mounting housing is provided with a second opening or a second groove; at the first position, the cover housing extends into the mounting housing from the second opening or the second groove, so as to be accommodated inside the mounting housing; or
a rear side of the mounting housing is provided with a second sleeving portion, and the cover housing is movably sleeved with the second sleeving portion; at the first position, the cover housing and the second sleeving portion move close to each other, so as to enable at least a portion of the cover housing to be accommodated inside the mounting housing; at the second position, the cover housing and the second sleeving portion move away from each other, so as to enable at least a portion of the cover housing to extend out of the mounting housing and cover the gap region.

12. The monitoring device according to any one of claims 2-5, **characterized in that**, one end of the cover housing is fixed to the mounting housing, and the other end of the cover housing is fixed to the device housing, wherein the cover housing is provided with a plurality of sub-housings which are movably connected in sequence, so as to cover the gap region and simultaneously allow the screen assembly to be tilted from the first position to the second position relative to the device body.

13. The monitoring device according to claim 12, **characterized in that**, the plurality of sub-housings are movably connected in sequence in a sleeved manner; wherein the cover housing has a state of moving close and a state of moving away; wherein in the state of moving close, the plurality of sub-housings are respectively accommodated inside an adjacent sub-housing in sequence; in the state of moving away, the plurality of sub-housings move away from one another and adjacent sub-housings maintain in connection with each other; wherein the screen assembly is configured to drive the cover housing to switch between the state of moving close and the state of moving away by tilting relative to the device body.

14. The monitoring device according to claim 13, **characterized in that**, the plurality of sub-housings comprise a first sub-housing with one end fixed to the mounting housing, a second sub-housing with one end fixed to the device housing, and a third sub-housing located between the first sub-housing and the second sub-housing; wherein the first sub-housing is provided with a first position-limiting portion; the second sub-housing is provided with a second position-limiting portion; the third sub-housing is provided with a third position-limiting portion on a side which is adjacent to the first sub-housing, and a fourth position-limiting portion on a side which is adjacent to the second sub-housing;
the third sub-housing is a single sub-housing, wherein when the screen assembly is tilted downward from the first position to the second position, the third position-limiting portion abuts against the first position-limiting portion, and the fourth position-limiting portion abuts against the second position-limiting portion; or
the third sub-housing comprises at least two sub-housings; wherein when the screen assembly is tilted downward from the first position to the second position, the third position-limiting portion of the third sub-housing, which is most adjacent to the first sub-housing, abuts against the first position-limiting portion; the fourth position-limiting portion of the third sub-housing, which is most adjacent to the second sub-housing, abuts against the second position-limiting portion; one of two adjacent sub-housings of the third sub-housing abuts against, through its third position-limiting portion, the fourth position-limiting portion of the other of said two adjacent sub-housings.

15. The monitoring device according to any one of claims 2-5, **characterized in that**:
a front side of the device housing is provided with a first opening or a first groove, a rear side of the mounting housing is provided with a second sleeving portion, wherein one end of the cover housing extends into the device housing from the first opening or the first groove, and the second sleeving portion is sleeved on the other end of the cover housing; or
a rear side of the mounting housing is provided with a second opening or a second groove, a front side of the device housing is provided with a first sleeving portion; wherein one end of the cover housing extends into the mounting housing from the second opening or the second groove, and the first sleeving portion is sleeved on the other end of the cover housing.

16. The monitoring device according to any one of claims 2-5, **characterized in that**, the cover housing is in rotatable connection with at least one of the mounting housing and the device housing.

17. The monitoring device according to any one of claims 2-5, **characterized in that**, opposite sides of the cover housing are respectively fixed to the mounting housing and the device housing, wherein the screen assembly is configured to drive the cover housing to switch between folding and unfolding by tilting relative to the device body.

18. The monitoring device according to claim 17, **characterized in that**, the cover housing comprises a foldable curtain and a support frame, wherein when the screen assembly is tilted from the first position to the second position, at least a portion of the screen assembly gradually moves away from the device body, the cover housing is unfolded and the support frame supports the foldable curtain.

19. The monitoring device according to claim 17, **characterized in that**, further comprising a support member for an opening angle, which is in respective rotatable connection with the device housing and the mounting housing; when the screen assembly is tilted from the first position to the second position, the support member for an opening angle supports between the device housing and the mounting housing.

20. The monitoring device according to claim 17, **characterized in that**, the cover housing comprises a plurality of foldable portions which are arranged in sequence, wherein a variable angle is formed between adjacent foldable portions; wherein while the screen assembly is tilted, an angle between adjacent foldable portions is gradually increased to unfold the cover housing, and an angle between adjacent foldable portions is gradually decreased to fold the cover housing.

21. The monitoring device according to claim 1, **characterized in that**, further comprising a cover housing, wherein at least one of the device housing, the mounting housing and the connection assembly is provided with a mounting portion of the cover housing for mounting the cover housing, when the cover housing is mounted at the mounting portion of the cover housing, the cover housing covers a gap region, which is formed between the device body and the screen assembly due to tilting of the screen assembly relative to the device body.

22. The monitoring device according to claim 21, **characterized in that**, when the cover housing is mounted at the mounting portion of the cover housing; while the screen assembly is tilted, an upper edge of the screen assembly gradually moves away from the device body, and the gap region, which is formed between the screen assembly and the device body, gradually increases.

23. The monitoring device according to claim 21 or 22, **characterized in that**, the cover housing is in detachable connection with the mounting portion of the cover housing.

24. The monitoring device according to claim 23, **characterized in that**, the mounting portion of the cover housing is one of a snap-fit, a plug-in slot, and a fastening connection hole.

25. The monitoring device according to claim 2 or 21, **characterized in that**:
the cover housing comprises an upper covering portion, and a side covering portion which extends downward from at least one of opposite ends of the upper covering portion; or
the cover housing is in a shape of an elongated strip or a shape of a ring.

26. The monitoring device according to claim 2 or 21, **characterized in that**, a region, which is covered by the cover housing, is at least 50% of the gap region.

27. The monitoring device according to claim 2 or 21, **characterized in that**:
the cover housing surrounds a periphery side of the gap region, so as to form an enclosed space between the device body and the screen assembly; or
the cover housing surrounds and seals a periphery side of the gap region, so as to form a sealed space between the device body and the screen assembly.

28. The monitoring device according to claim 1, **characterized in that**, the connection assembly comprises a fixing block which is fixed to the device housing, and a rotating block which is fixed to the mounting housing, wherein the rotating block is in rotatable connection with the fixing block.

29. The monitoring device according to claim 28, **characterized in that**, when the screen assembly is tilted downward to the second position, the rotating block abuts against the device housing.

30. The monitoring device according to claim 29, **characterized in that**, the fixing block is provided with a mounting opening, the rotating block rotatably penetrates through and is arranged inside the mounting opening; wherein the rotating block and a region of the device housing, which region is located at the mounting opening, are arranged with a gap therebetween, which gap forms a space for the rotating block to rotate therein.

31. The monitoring device according to any one of claims 1-5, **characterized in that**, the connection assembly comprises a damping shaft and a connection column which is provided with a shaft hole, wherein one of the damping shaft and the connection column is fixed to the device housing, and the other of the damping shaft and the connection column is fixed to the mounting housing, wherein the damping shaft rotatably penetrates through and is arranged inside the shaft hole.

32. The monitoring device according to claim 31, **characterized in that**, a side wall of the connection column is provided with a position-limiting groove, which extends along an axial direction and is connected with the shaft hole; wherein the damping shaft comprises a shaft body and a position-limiting plate which is arranged at a side wall of the shaft body, the shaft body rotatably penetrates through and is arranged inside the shaft hole, the position-limiting plate penetrates through the position-limiting groove, so as to extend out of the connection column; when the screen assembly is tilted to the first position or the second position, the position-limiting plate abuts against the connection column.

33. The monitoring device according to claim 1, **characterized in that**, the connection assembly comprises a driving motor and a transmission mechanism, wherein the driving motor is configured to drive the transmission mechanism to move, so as to enable the transmission mechanism to drive the screen assembly to tilt.

34. The monitoring device according to claim 33, **characterized in that**, the transmission mechanism comprises a connection shaft, an adapter plate, a worm wheel and a worm rod, wherein the worm wheel penetrates through and is arranged at the connection shaft, the worm rod is meshed with the worm wheel, the adapter plate is in respective connection with the connection shaft and the mounting housing, the driving motor is in drive connection with the worm rod.

35. The monitoring device according to claim 34, **characterized in that**, the transmission mechanism further comprises a driving rod which is meshed with the worm rod, wherein the driving motor is connected with the driving rod to drive the driving rod to rotate, so as to further drive the worm rod to rotate.

36. The monitoring device according to claim 1, **characterized in that**, further comprising a position-restricting structure, which is configured to restrict the screen assembly to the first position; wherein when the screen assembly is released from restriction of the position-restricting structure, the screen assembly is capable of being tilted to the second position.

37. The monitoring device according to claim 36, **characterized in that**, when the screen assembly is released from the restriction of the position-restricting structure, the connection assembly pushes the screen assembly to tilt to the second position.

38. The monitoring device according to claim 37, **characterized in that**, the connection assembly comprises a pushing member and a second elastic member, when the screen assembly is released from the restriction of the position-restricting structure, the pushing member pushes the screen assembly to tilt to the second position under an elastic force of the second elastic member.

39. The monitoring device according to claim 38, **characterized in that**, the pushing member comprises a rod portion and a pushing portion; wherein the rod portion is rotatably arranged at the device housing, the pushing portion is arranged on a side of the rod portion and faces the screen assembly, the second elastic member is arranged on a side of the rod portion which side is away from the pushing portion, the pushing member is rotated under the elastic force of the second elastic member, so as to push the screen assembly to tilt.

40. The monitoring device according to claim 36, **characterized in that**, the position-restricting structure comprises a first locking member and a second locking member, the first locking member is movably arranged at one of the screen assembly and the device body, the second locking member is arranged at the other of the screen assembly and the device body, the first locking member is locked and unlocked with the second locking member through a movement of the first locking member.

41. The monitoring device according to claim 40, **characterized in that**, the first locking member and the second locking member are locked with each other by abutting against each other; wherein the first locking member is switched to an unlocking state in which the first locking member and the second locking member are separated from each other, through the first locking member rotating relative to the second locking member under an action of an external force.

42. The monitoring device according to claim 36, **characterized in that**, the screen assembly is released from the restriction of the position-restricting structure under a push of an external force.

43. The monitoring device according to claim 42, **characterized in that**:
the position-restricting structure comprises a magnetic member, which is fixed to one of the screen assembly and the device body, wherein the other of the screen assembly and the device body is fitted with the magnetic member through magnetic attraction; or
the position-restricting structure comprises a first magnetic member and a second magnetic member which are fitted with each other through magnetic attraction; wherein the first magnetic member is fixed to the screen assembly, and the second magnetic member is fixed to the device body.

44. The monitoring device according to claim 1, **characterized in that**, the connection assembly comprises a connection frame, a rotation shaft, a damping plate, a fixing member and an adjustment nut, wherein the rotation shaft is in fixing connection with the connection frame, the fixing member and the damping plate penetrate through and are arranged at the rotation shaft, the fixing member is capable of rotating relative to the rotation shaft; wherein the adjustment nut is located on a side of the damping plate which side is away from the fixing member, and the adjustment nut is in threaded connection with the rotation shaft;
the connection frame is in fixing connection with one of the mounting housing and the device housing, and the fixing member is in fixing connection with the other of the mounting housing and the device housing.

45. The monitoring device according to claim 44, **characterized in that**, the fixing member is provided with a fifth position-limiting portion, the connection assembly further comprises a second position-limiting member which is fixed to the rotation shaft and located on a side of the fixing member; wherein when the fixing member is rotated to the first position or the second position, the fifth position-limiting portion abuts against the second position-limiting member.

46. The monitoring device according to claim 1, **characterized in that**, the connection assembly is a hinge which is in respective fixing connection with the screen assembly and the device body.

47. The monitoring device according to claim 46, **characterized in that**, the hinge comprises a fixing arm and a connection arm, wherein the fixing arm is fixed to the device body, one end of the connection arm is in rotatable connection with the fixing arm, and the other end of the connection arm is in fixing connection with the mounting housing.

48. The monitoring device according to claim 1, **characterized in that**, the connection assembly comprises a first slidable portion which is arranged at the device housing and a second slidable portion which is arranged at the mounting housing; wherein the second slidable portion is slidably fitted with the first slidable portion, so as to enable the screen assembly to be tilted relative to the device body by sliding.

49. The monitoring device according to claim 25, **characterized in that**:
the first slidable portion is arc-shaped; or
the first slidable portion is a slide rail, and the second slidable portion is a slidable member; or
the first slidable portion is a slide groove, and the second slidable portion is a slidable member which extends into the slide groove.

50. The monitoring device according to claim 1, **characterized in that**, the connection assembly comprises a plurality of connection rods, each connection rod is in respective rotatable connection with the mounting housing and the device housing.

51. The monitoring device according to claim 50, **characterized in that**, a number of the connection rods is four, wherein two of the four connection rods are respectively arranged on opposite sides of the device housing along a horizontal direction, wherein the two connection rods, which are located on a same side of the device housing, are respectively arranged on a top side and a bottom side of the device housing.

52. The monitoring device according to claim 1, **characterized in that**, a partial region of a bottom side of the device housing protrudes from a front side of the device housing to form a convex portion, wherein the screen assembly is arranged on an upper side of the convex portion.

53. The monitoring device according to claim 52, **characterized in that**, the screen assembly is tilted about a tilting axis; a size of a space which is formed between the screen assembly and the convex portion changes with tilting of the screen assembly; at least when the screen assembly is at the first position or the second position, a cleanable space is capable of being formed between the screen assembly and the convex portion.

54. The monitoring device according to claim 52, **characterized in that**, the screen assembly is tilted about a tilting axis, the connection assembly is arranged at the convex portion.

55. The monitoring device according to claim 54, **characterized in that**, the connection assembly is a hinge which is arranged at a front end of the convex portion.

56. The monitoring device according to claim 1, **characterized in that**, the screen assembly is tilted about a tilting axis, a partial region of the device housing protrudes from a front side of the device housing, so as to form a connection portion above the ground, wherein the connection assembly is arranged at the connection portion.

57. The monitoring device according to claim 56, **characterized in that**, when the screen assembly is tilted downward from the first position to the second position, a distance between a horizontal center line of the connection portion and a horizontal center line of the monitoring device is respectively less than a distance between the horizontal center line of the connection portion and a top surface of the monitoring device and a distance between the horizontal center line of the connection portion and a bottom surface of the monitoring device, wherein a lower side of the connection portion is provided with a movement space for the screen assembly to be tilted to the second position.

58. The monitoring device according to claim 1, **characterized in that**, the device body comprises a base which is arranged at a bottom of the device housing.

59. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a mounting housing and a screen which is arranged at the mounting housing;
a connection assembly, wherein the screen assembly is in movable connection with the device body through the connection assembly, so as to enable the screen assembly to be tilted downward from a first position to a second position relative to the device body to exhibit a posture with an angle of depression; wherein when the screen assembly is tilted downward from the first position to the second position, an upper portion of the screen assembly gradually moves away from the device body or a lower portion of the screen assembly gradually moves close to the device body.

60. The monitoring device according to claim 59, **characterized in that**, a maximum of the angle of depression of the screen assembly is 45°.

61. The monitoring device according to claim 29, **characterized in that**, further comprising a cover housing; wherein the cover housing is arranged between the device housing and the mounting housing, or is arranged at at least one of the device housing, the mounting housing and the connection assembly, so as to cover a gap region, which is formed between the device body and the screen assembly due to downward tilting of the screen assembly relative to the device body.

62. The monitoring device according to claim 61, **characterized in that**, at the second position, the cover housing surrounds, and encloses or seals the gap region.

63. The monitoring device according to claim 61, **characterized in that**:
the cover housing and the device housing are integrally formed; or
the cover housing and the mounting housing are integrally formed.

64. The monitoring device according to claim 59, **characterized in that**, further comprising a cover housing, wherein at least one of the device housing, the mounting housing and the connection assembly is provided with a mounting portion of the cover housing for mounting the cover housing, when the cover housing is mounted at the mounting portion of the cover housing, the cover housing covers a gap region, which is formed between the device body and the screen assembly due to downward tilting of the screen assembly relative to the device body.

65. The monitoring device according to claim 59, **characterized in that**:
the cover housing comprises an upper covering portion, and a side covering portion which extends downward from at least one of opposite ends of the upper covering portion; or
the cover housing is in a shape of an elongated strip or a shape of a ring.

66. The monitoring device according to claim 59, **characterized in that**, the cover housing is in a shape of a rectangle ring.

67. The monitoring device according to claim 59, **characterized in that**, further comprising a position-restricting structure, which is configured to restrict the screen assembly to the first position; wherein when the screen assembly is released from restriction of the position-restricting structure, the screen assembly is capable of being tilted to the second position.

68. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a mounting housing and a screen which is arranged at the mounting housing;
a connection assembly, wherein the screen assembly is in movable connection with the device housing through the connection assembly, so as to enable the screen assembly to be tilted downward from an initial position relative to the device body to form a posture with an angle of depression; wherein when the screen assembly is tilted downward from the initial position, the entirety of the monitoring device maintains stability.

69. The monitoring device according to claim 68, **characterized in that**, while the screen assembly is tilted downward from the initial position, a projection for a center of gravity of the monitoring device on a horizontal plane is always within a projection range of a support region, which supports the device body, on the horizontal plane.

70. The monitoring device according to claim 68 or 69, **characterized in that**, while the screen assembly is tilted downward from the initial position, the monitoring device does not fall forward; and/or a bottom surface of the monitoring device, which bottom surface is in contact with a support surface which supports the device body, is always in contact with the support surface.

71. The monitoring device according to claim 68 or 69, **characterized in that**:
a bottom surface of the device body is configured to form a continuous support contact surface, and a region which is enclosed by an outer contour of the support contact surface is a support region; or
a plurality of support legs are arranged at a bottom of the device housing, wherein outer contours of bottom surfaces of the support legs are connected in sequence by straight lines, and a largest region enclosed by the outer contours and the straight lines is a support region.

72. The monitoring device according to claim 68 or 69, **characterized in that**, a maximum of the angle of depression of the screen assembly is 45°.

73. The monitoring device according to claim 68 or 69, **characterized in that**, a distance between a tilting axis of the screen assembly relative to the device body and a horizontal center line of the monitoring device is respectively less than a distance between the tilting axis and a top surface of the monitoring device and a distance between the tilting axis and a bottom surface of the monitoring device.

74. The monitoring device according to claim 68 or 69, **characterized in that**, the device body comprises a base which is arranged at a bottom of the device housing.

75. The monitoring device according to claim 68 or 69, **characterized in that**, a partial region of a bottom side of the device housing protrudes from a front side of the device housing to form a convex portion, wherein the screen assembly is arranged on an upper side of the convex portion.

76. The monitoring device according to claim 75, **characterized in that**:
the connection assembly is arranged at the convex portion; or
a partial region of the device housing protrudes from the front side of the device housing, so as to form a connection portion which is located on an upper side of the convex portion, and the connection assembly is arranged at the connection portion.

77. The monitoring device according to claim 68 or 69, **characterized in that**, when the screen assembly forms the posture with an angle of depression, an upper edge of the screen assembly gradually moves away from the device body or a lower edge of the screen assembly gradually moves away from the device body, a gap region is formed between the screen assembly and the device body;
the monitoring device further comprises a cover housing, which is arranged between the device housing and the mounting housing or at one of the device housing, the mounting housing and the connection assembly, so as to cover the gap region.

78. A medical device, **characterized in that**, comprising:
a device body, which comprises a device housing;
a screen assembly, which comprises a mounting housing and a screen which is arranged at the mounting housing; wherein the screen assembly is in movable connection with the device body and is capable of being tilted downward from a first position to a second position relative to the device body to exhibit a posture with an angle of depression, wherein when the screen assembly is tilted from the first position to the second position, an upper edge of the screen assembly gradually moves away from the device body or a lower edge of the screen assembly gradually moves away from the device body, so as to form a gap region between the screen assembly and the device body;
a cover housing, which is located between the device body and the screen assembly, so as to cover the gap region.

79. The medical device according to claim 78, **characterized in that**:
both ends of the cover housing are respectively arranged at the device housing and the mounting housing; or
one end of the cover housing is arranged at one of the device housing and the mounting housing; or
the medical device further comprises a connection assembly, wherein the screen assembly is in movable connection with the device body through the connection assembly, so as to enable the screen assembly to be tilted from the first position to the second position relative to the device body, wherein one end of the cover housing is arranged at the connection assembly.

80. The medical device according to claim 79, **characterized in that**, further comprising a connection assembly, wherein the connection assembly is in rotatable connection with the cover housing, and the mounting housing is fixed to the connection assembly.

81. The medical device according to claim 78 or 79, **characterized in that**, when the screen assembly is at the second position, the cover housing surrounds, and encloses or seals the gap region.

82. The medical device according to claim 78 or 79, **characterized in that**, one end of the cover housing is fixed to the mounting housing; at the first position, the cover housing is accommodated inside the device housing, and, at the second position, at least a portion of the cover housing extends out of the device housing and covers the gap region.

83. The medical device according to claim 82, **characterized in that**:
a front side of the device housing is provided with a first opening or a first groove; at the first position, the cover housing extends into the device housing from the first opening or the first groove, so as to be accommodated inside the device housing; or
a front side of the device housing is provided with a first sleeving portion, and the cover housing is movably sleeved with the first sleeving portion; at the first position, the cover housing and the first sleeving portion move close to each other, so as to enable at least a portion of the cover housing to be accommodated inside the device housing; at the second position, the cover housing and the first sleeving portion move away from each other, so as to enable at least a portion of the cover housing to extend out of the device housing and cover the gap region.

84. The medical device according to claim 82, **characterized in that**, the screen assembly is tilted downward from the first position to the second position, the cover housing is provided with a sixth position-limiting portion; wherein at least a portion of the cover housing, which portion is provided with the sixth position-limiting portion, extends into the device housing;
the medical device further comprises a position-limiting structure which is arranged on an inner wall of the device housing; wherein the position-limiting structure comprises a first elastic member and a first position-limiting member, wherein the first elastic member is configured to enable the first position-limiting member to avoid the cover housing by retracting while the cover housing is mounted, so as to enable the sixth position-limiting portion to move to a rear side of the position-limiting structure; when the screen assembly is tilted downward from the first position to the second position, the first position-limiting member abuts against the sixth position-limiting portion.

85. The medical device according to claim 78 or 79, **characterized in that**, one end of the cover housing is fixed to the device housing; at the first position, at least a portion of the cover housing is accommodated inside the mounting housing, and, at the second position, at least a portion of the cover housing extends out of the mounting housing and covers the gap region.

86. The medical device according to claim 85, **characterized in that**:
a rear side of the mounting housing is provided with a second opening or a second groove; at the first position, the cover housing extends into the mounting housing from the second opening or the second groove, so as to be accommodated inside the mounting housing; or
a rear side of the mounting housing is provided with a second sleeving portion, and the cover housing is movably sleeved with the second sleeving portion; at the first position, the cover housing and the second sleeving portion move close to each other, so as to enable at least a portion of the cover housing to be accommodated inside the mounting housing; at the second position, the cover housing and the second sleeving portion move away from each other, so as to enable at least a portion of the cover housing to extend out of the mounting housing and cover the gap region.

87. The medical device according to claim 78 or 79, **characterized in that**, one end of the cover housing is fixed to the mounting housing, and the other end of the cover housing is fixed to the device housing, wherein the cover housing is provided with a plurality of sub-housings which are movably connected in sequence, so as to cover the gap region and simultaneously allow the screen assembly to be tilted from the first position to the second position relative to the device body.

88. The medical device according to claim 87, **characterized in that**, the plurality of sub-housings are movably connected in sequence in a sleeved manner; wherein the cover housing has a state of moving close and a state of moving away; wherein in the state of moving close, the plurality of sub-housings are respectively accommodated inside an adjacent sub-housing in sequence; in the state of moving away, the plurality of sub-housings move away from one another and adjacent sub-housings maintain in connection with each other; wherein the screen assembly is configured to drive the cover housing to switch between the state of moving close and the state of moving away by tilting relative to the device body.

89. The medical device according to claim 88, **characterized in that**, the plurality of sub-housings comprise a first sub-housing with one end fixed to the mounting housing, a second sub-housing with one end fixed to the device housing, and a third sub-housing located between the first sub-housing and the second sub-housing; wherein the first sub-housing is provided with a first position-limiting portion; the second sub-housing is provided with a second position-limiting portion; the third sub-housing is provided with a third position-limiting portion on a side which is adjacent to the first sub-housing, and a fourth position-limiting portion on a side which is adjacent to the second sub-housing;
the third sub-housing is a single sub-housing, wherein when the screen assembly is tilted downward from the first position to the second position, the third position-limiting portion abuts against the first position-limiting portion, and the fourth position-limiting portion abuts against the second position-limiting portion; or
the third sub-housing comprises at least two sub-housings; wherein when the screen assembly is tilted downward from the first position to the second position, the third position-limiting portion of the third sub-housing, which is most adjacent to the first sub-housing, abuts against the first position-limiting portion; the fourth position-limiting portion of the third sub-housing, which is most adjacent to the second sub-housing, abuts against the second position-limiting portion; one of two adjacent sub-housings of the third sub-housing abuts against, through its third position-limiting portion, the fourth position-limiting portion of the other of said two adjacent sub-housings.

90. The medical device according to claim 78 or 79, **characterized in that**:
a front side of the device housing is provided with a first opening or a first groove, a rear side of the mounting housing is provided with a second sleeving portion, wherein one end of the cover housing extends into the device housing from the first opening or the first groove, and the second sleeving portion is sleeved on the other end of the cover housing; or
a rear side of the mounting housing is provided with a second opening or a second groove, a front side of the device housing is provided with a first sleeving portion; wherein one end of the cover housing extends into the mounting housing from the second opening or the second groove, and the first sleeving portion is sleeved on the other end of the cover housing.

91. The medical device according to claim 78 or 79, **characterized in that**, the cover housing is in rotatable connection with at least one of the mounting housing and the device housing.

92. The medical device according to claim 78 or 79, **characterized in that**, opposite sides of the cover housing are respectively fixed to the mounting housing and the device housing, wherein the screen assembly is configured to drive the cover housing to switch between folding and unfolding by tilting relative to the device body.

93. The medical device according to claim 92, **characterized in that**, the cover housing comprises a foldable curtain and a support frame, wherein when the screen assembly is tilted from the first position to the second position, at least a portion of the screen assembly gradually moves away from the device body, the cover housing is unfolded and the support frame supports the foldable curtain.

94. The medical device according to claim 93, **characterized in that**, further comprising a support member for an opening angle, which is in respective rotatable connection with the device housing and the mounting housing; when the screen assembly is tilted from the first position to the second position, the support member for an opening angle supports between the device housing and the mounting housing.

95. The medical device according to claim 92, **characterized in that**, the cover housing comprises a plurality of foldable portions which are arranged in sequence, wherein a variable angle is formed between adjacent foldable portions; wherein while the screen assembly is tilted, an angle between adjacent foldable portions is gradually increased to unfold the cover housing, and an angle between adjacent foldable portions is gradually decreased to fold the cover housing.

96. The medical device according to claim 79, **characterized in that**, at least one of the device housing, the mounting housing and the connection assembly is provided with a mounting portion of the cover housing for mounting the cover housing, when the cover housing is mounted at the mounting portion of the cover housing, the cover housing covers a gap region, which is formed between the device body and the screen assembly due to tilting of the screen assembly relative to the device body.

97. The medical device according to claim 96, **characterized in that**, when the cover housing is mounted at the mounting portion of the cover housing; while the screen assembly is tilted, an upper edge of the screen assembly gradually moves away from the device body, and the gap region, which is formed between the screen assembly and the device body, gradually increases, and the cover housing always covers the gap region.

98. The medical device according to claim 96 or 97, **characterized in that**, the cover housing is in detachable connection with the mounting portion of the cover housing.

99. The medical device according to claim 98, **characterized in that**, the mounting portion of the cover housing is one of a snap-fit, a plug-in slot, and a fastening connection hole.

100. The medical device according to claim 78 or 79, **characterized in that**:
the cover housing comprises an upper covering portion, and a side covering portion which extends downward from at least one of opposite ends of the upper covering portion; or
the cover housing is in a shape of an elongated strip or a shape of a ring.

101. The medical device according to claim 78 or 79, **characterized in that**, the cover housing is in a shape of a rectangle ring.

102. The medical device according to claim 78 or 79, **characterized in that**, a region, which is covered by the cover housing, is at least 50% of the gap region.

103. The medical device according to claim 78 or 79, **characterized in that**:
the cover housing surrounds a periphery side of the gap region, so as to form an enclosed space between the device body and the screen assembly; or
the cover housing surrounds and seals a periphery side of the gap region, so as to form a sealed space between the device body and the screen assembly.

104. The medical device according to claim 78, **characterized in that**, further comprising a connection assembly, wherein the screen assembly is in movable connection with the device body through the connection assembly, so as to enable the screen assembly to be tilted from the first position to the second position relative to the device body.

105. The medical device according to claim 104, **characterized in that**, the connection assembly comprises a fixing block which is fixed to the device housing, and a rotating block which is fixed to the mounting housing, wherein the rotating block is in rotatable connection with the fixing block.

106. The medical device according to claim 105, **characterized in that**, when the screen assembly is tilted downward to the second position, the rotating block abuts against the device housing.

107. The medical device according to claim 106, **characterized in that**, the fixing block is provided with a mounting opening, the rotating block rotatably penetrates through and is arranged inside the mounting opening; wherein the rotating block and a region of the device housing, which region is located at the mounting opening, are arranged with a gap therebetween, which gap forms a space for the rotating block to rotate therein.

108. The medical device according to claim 104, **characterized in that**, the connection assembly comprises a damping shaft and a connection column which is provided with a shaft hole, wherein one of the damping shaft and the connection column is fixed to the device housing, and the other of the damping shaft and the connection column is fixed to the mounting housing, wherein the damping shaft rotatably penetrates through and is arranged inside the shaft hole.

109. The medical device according to claim 108, **characterized in that**, a side wall of the connection column is provided with a position-limiting groove, which extends along an axial direction and is connected with the shaft hole; wherein the damping shaft comprises a shaft body and a position-limiting plate which is arranged at a side wall of the shaft body, the shaft body rotatably penetrates through and is arranged inside the shaft hole, the position-limiting plate penetrates through the position-limiting groove, so as to extend out of the connection column; when the screen assembly is tilted to the first position or the second position, the position-limiting plate abuts against the connection column.

110. The medical device according to claim 104, **characterized in that**, the connection assembly comprises a driving motor and a transmission mechanism, wherein the driving motor is configured to drive the transmission mechanism to move, so as to enable the transmission mechanism to drive the screen assembly to tilt.

111. The medical device according to claim 110, **characterized in that**, the transmission mechanism comprises a connection shaft, an adapter plate, a worm wheel and a worm rod, wherein the worm wheel penetrates through and is arranged at the connection shaft, the worm rod is meshed with the worm wheel, the adapter plate is in respective connection with the connection shaft and the mounting housing, the driving motor is in drive connection with the worm rod.

112. The medical device according to claim 111, **characterized in that**, the transmission mechanism further comprises a driving rod which is meshed with the worm rod, wherein the driving motor is connected with the driving rod to drive the driving rod to rotate, so as to further drive the worm rod to rotate.

113. The medical device according to claim 104, **characterized in that**, further comprising a position-restricting structure, which is configured to restrict the screen assembly to the first position; wherein when the screen assembly is released from restriction of the position-restricting structure, the screen assembly is capable of being tilted to the second position.

114. The medical device according to claim 113, **characterized in that**, when the screen assembly is released from the restriction of the position-restricting structure, the connection assembly pushes the screen assembly to tilt to the second position.

115. The medical device according to claim 114, **characterized in that**, the connection assembly comprises a pushing member and a second elastic member, when the screen assembly is released from the restriction of the position-restricting structure, the pushing member pushes the screen assembly to tilt to the second position under an elastic force of the second elastic member.

116. The medical device according to claim 115, **characterized in that**, the pushing member comprises a rod portion and a pushing portion; wherein the rod portion is rotatably arranged at the device housing, the pushing portion is arranged on a side of the rod portion and faces the screen assembly, the second elastic member is arranged on a side of the rod portion which side is away from the pushing portion, the pushing member is rotated under the elastic force of the second elastic member, so as to push the screen assembly to tilt.

117. The medical device according to claim 113, **characterized in that**, the position-restricting structure comprises a first locking member and a second locking member, the first locking member is movably arranged at one of the screen assembly and the device body, the second locking member is arranged at the other of the screen assembly and the device body, the first locking member is locked and unlocked with the second locking member through a movement of the first locking member.

118. The medical device according to claim 117, **characterized in that**, the first locking member and the second locking member are locked with each other by abutting against each other; wherein the first locking member is switched to an unlocking state in which the first locking member and the second locking member are separated from each other, through the first locking member rotating relative to the second locking member under an action of an external force.

119. The medical device according to claim 113, **characterized in that**, the screen assembly is released from the restriction of the position-restricting structure under a push of an external force.

120. The medical device according to claim 119, **characterized in that**:
the position-restricting structure comprises a magnetic member, which is fixed to one of the screen assembly and the device body, wherein the other of the screen assembly and the device body is fitted with the magnetic member through magnetic attraction; or
the position-restricting structure comprises a first magnetic member and a second magnetic member which are fitted with each other through magnetic attraction; wherein the first magnetic member is fixed to the screen assembly, and the second magnetic member is fixed to the device body.

121. The medical device according to claim 104, **characterized in that**, the connection assembly comprises a connection frame, a rotation shaft, a damping plate, a fixing member and an adjustment nut, wherein the rotation shaft is in fixing connection with the connection frame, the fixing member and the damping plate penetrate through and are arranged at the rotation shaft, the fixing member is capable of rotating relative to the rotation shaft; wherein the adjustment nut is located on a side of the damping plate which side is away from the fixing member, and the adjustment nut is in threaded connection with the rotation shaft;
the connection frame is in fixing connection with one of the mounting housing and the device housing, and the fixing member is in fixing connection with the other of the mounting housing and the device housing.

122. The medical device according to claim 121, **characterized in that**, the fixing member is provided with a fifth position-limiting portion, the connection assembly further comprises a second position-limiting member which is fixed to the rotation shaft and located on a side of the fixing member; wherein when the fixing member is rotated to the first position or the second position, the fifth position-limiting portion abuts against the second position-limiting member.

123. The medical device according to claim 104, **characterized in that**, the connection assembly is a hinge which is in respective fixing connection with the screen assembly and the device body.

124. The medical device according to claim 123, **characterized in that**, the hinge comprises a fixing arm and a connection arm, wherein the fixing arm is fixed to the device body, one end of the connection arm is in rotatable connection with the fixing arm, and the other end of the connection arm is in fixing connection with the mounting housing.

125. The medical device according to claim 104, **characterized in that**, the connection assembly comprises a first slidable portion which is arranged at the device housing and a second slidable portion which is arranged at the mounting housing; wherein the first slidable portion is arc-shaped; wherein the second slidable portion is slidably fitted with the first slidable portion, so as to enable the screen assembly to be tilted relative to the device body by sliding.

126. The medical device according to claim 119, **characterized in that**:
the first slidable portion is a slide rail, and the second slidable portion is a slidable member; or
the first slidable portion is a slide groove, and the second slidable portion is a slidable member which extends into the slide groove.

127. The medical device according to claim 104, **characterized in that**, the connection assembly comprises a plurality of connection rods, each connection rod is in respective rotatable connection with the mounting housing and the device housing.

128. The medical device according to claim 127, **characterized in that**, a number of the connection rods is four, wherein two of the four connection rods are respectively arranged on opposite sides of the device housing along a horizontal direction, wherein the two connection rods, which are located on a same side of the device housing, are respectively arranged on a top side and a bottom side of the device housing.

129. The medical device according to claim 78 or 79, **characterized in that**, a partial region of a bottom side of the device housing protrudes from a front side of the device housing to form a convex portion, wherein the screen assembly is arranged on an upper side of the convex portion.

130. The medical device according to claim 129, **characterized in that**, the screen assembly is tilted about a tilting axis; a size of a space which is formed between the screen assembly and the convex portion changes with tilting of the screen assembly; at least when the screen assembly is at the first position or the second position, a cleanable space is capable of being formed between the screen assembly and the convex portion.

131. The medical device according to claim 100, **characterized in that**, the connection assembly is arranged at the convex portion.

132. The medical device according to claim 131, **characterized in that**, the connection assembly is a hinge which is arranged at a front end of the convex portion.

133. The medical device according to claim 104, **characterized in that**, the screen assembly is tilted about a tilting axis, a partial region of the device housing protrudes from a front side of the device housing, so as to form a connection portion above the ground, wherein the connection assembly is arranged at the connection portion.

134. The medical device according to claim 133, **characterized in that**, the screen assembly is tilted downward from the first position to the second position, a distance between a horizontal center line of the connection portion and a horizontal center line of the medical device is respectively less than a distance between the horizontal center line of the connection portion and a top surface of the medical device and a distance between the horizontal center line of the connection portion and a bottom surface of the medical device, wherein a lower side of the connection portion is provided with a movement space for the screen assembly to be tilted to the second position.

135. The medical device according to claim 78 or 79, **characterized in that**, the cover housing is a flexible structure.

136. The medical device according to claim 78 or 79, **characterized in that**, the medical device is a monitoring device, which comprises:
a main control board which is arranged inside the device housing or the mounting housing, wherein a processor is arranged on the main control board;
a parameter sensor interface, which is arranged inside the device housing or the mounting housing and connected with the processor, wherein the parameter sensor interface is connected with a parameter sensor to obtain physiological parameter data of a patient acquired by the parameter sensor; wherein the processor is configured to obtain the physiological parameter data of the patient acquired by the parameter sensor via the parameter sensor interface, and process the physiological parameter data;
a display, which is connected with the processor, wherein the processor is configured to control the display to display processed physiological parameter data of the patient.

137. The medical device according to claim 78 or 79, **characterized in that**, the medical device is a monitoring device, which comprises:
a main control board which is arranged inside the device housing or the mounting housing, wherein a processor is arranged on the main control board;
a communication interface board, which is arranged inside the device housing or the mounting housing, and is configured to obtain, from an external plug-in, physiological parameter data of a patient acquired by a parameter sensor; wherein the processor is configured to obtain the physiological parameter data of the patient acquired by the parameter sensor and then transmitted by the communication interface board, and process the physiological parameter data;
a display, which is connected with the processor, wherein the processor is configured to control the display to display processed physiological parameter data of the patient.

138. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a mounting housing and a screen which is arranged at the mounting housing; wherein the screen assembly is in movable connection with the device body and is capable of being tilted from a first position to a second position relative to the device body; wherein when the screen assembly is tilted from the first position to the second position, at least a portion of the screen assembly gradually moves away from or close to the device body;
a position-restricting structure, which is configured to restrict the screen assembly to the first position, the second position, or at least one intermediate position between the first position and the second position, so as to restrict the screen assembly from rotating relative to the device body.

139. The monitoring device according to claim 138, **characterized in that**, the position-restricting structure comprises a first locking member and a second locking member, the first locking member is movably arranged at one of the screen assembly and the device body, the second locking member is arranged at the other of the screen assembly and the device body, the first locking member is locked and unlocked with the second locking member through a movement of the first locking member.

140. The monitoring device according to claim 139, **characterized in that**, the first locking member and the second locking member are locked with each other by abutting against each other; wherein the first locking member is switched to an unlocking state in which the first locking member and the second locking member are separated from each other, through the first locking member rotating relative to the second locking member under an action of an external force.

141. The monitoring device according to claim 138, **characterized in that**, the screen assembly is released from the restriction of the position-restricting structure under a push of an external force.

142. The monitoring device according to claim 141, **characterized in that**:
the position-restricting structure comprises a magnetic member, which is fixed to one of the screen assembly and the device body, wherein the other of the screen assembly and the device body is fitted with the magnetic member through magnetic attraction; or
the position-restricting structure comprises a first magnetic member and a second magnetic member which are fitted with each other through magnetic attraction; wherein the first magnetic member is fixed to the screen assembly, and the second magnetic member is fixed to the device body.

143. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
wherein, the monitoring device is provided with a convex portion which protrudes forward from a bottom side of the device housing;
a screen assembly, which comprises a mounting housing and a screen which is arranged at the mounting housing; wherein the screen assembly is in movable connection with the device body and is capable of being tilted from a first position to a second position relative to the device body;
wherein, a lower edge of the screen assembly is located above the convex portion.

144. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a support portion, a mounting housing, and a screen which is arranged at the mounting housing; wherein the screen assembly is in movable connection with the device body and is capable of being tilted downward from an initial position to an extreme position relative to the device body to form a posture with an angle of depression; wherein, the support portion supports the screen assembly at at least one position, while the screen assembly is tilted downward from the initial position to the extreme position.

145. The monitoring device according to claim 143 or 144, **characterized in that**, an upper surface of the convex portion have a shape, which is capable of accommodating with a movement of a lower edge of the screen assembly, while the screen assembly is tilted from the initial position to the extreme position.

146. The monitoring device according to claim 145, **characterized in that**, the upper surface of the convex portion is inclined downward from a rear side to a front side.

147. The monitoring device according to claim 144, **characterized in that**, a size of a space which is formed between the screen assembly and the convex portion changes with tilting of the screen assembly; at least when the screen assembly is at the initial position or the extreme position, a cleanable space is capable of being formed between the screen assembly and the convex portion.

148. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a mounting housing and a screen which is arranged at the mounting housing;
a connection assembly, which is mounted on a front side of the device housing; wherein the screen assembly is in movable connection with the device body through the connection assembly, so as to enable the screen assembly to be tilted downward from an initial position to an extreme position relative to the device body;
wherein, while the screen assembly is tilted downward from the initial position to the extreme position, a distance between an upper edge of the screen assembly and the device body gradually increases, and a distance between a lower edge of the screen assembly and the device body gradually decreases; wherein when the screen assembly is tilted to the extreme position, the screen assembly is in contact with the device housing or a gap is formed between the screen assembly and the device housing.

149. The monitoring device according to claim 148, **characterized in that**, the connection assembly is mounted on the front side of the device housing in a manner that at least a portion of the connection assembly protrudes outward from the front side of the device housing for a first distance, so as to enable a lower side of the screen assembly to have a sufficient movement space while the screen assembly is tilted downward from the initial position to the extreme position.

150. The monitoring device according to claim 148, **characterized in that**, the device housing has a shape that enables a lower side of the screen assembly to have a sufficient movement space while the screen assembly is tilted downward from the initial position to the extreme position.

151. The monitoring device according to any one of claims 148-150, **characterized in that**, the screen assembly is tilted about a tilting axis, a partial region of the device housing protrudes from the front side of the device housing, so as to form a connection portion above the ground, wherein the connection assembly is arranged at the connection portion.

152. The monitoring device according to claim 151, **characterized in that**, further comprising a convex portion, which protrudes forward from a bottom side of the device housing and is located on a lower side of the screen assembly; wherein a size of a space which is formed between the screen assembly and the convex portion changes with tilting of the screen assembly; at least when the screen assembly is at the initial position or the extreme position, a cleanable space is capable of being formed between the screen assembly and the convex portion.

153. The monitoring device according to any one of claims 148-150, **characterized in that**, on a projection plane which is perpendicular to a tilting axis of the screen assembly, at least a portion of a contour line of the front side of the device housing is inclined toward a rear side of the device housing from top to bottom.

154. The monitoring device according to any one of claims 148-150, **characterized in that**, at least a portion of the front side of the device housing is recessed toward a rear side of the device housing, so as to form a movement space for a lower side of the screen assembly.

155. A medical device, **characterized in that**, comprising:
a device body, which comprises a device housing;
a screen assembly, which comprises a mounting housing and a screen which is arranged at the mounting housing;
a connection assembly, wherein the screen assembly is in movable connection with the device body through the connection assembly; wherein the connection assembly comprises a first slidable portion and a second slidable portion which are slidably fitted with each other; wherein the first slidable portion is arranged at the device housing and the second slidable portion is arranged at the mounting housing, so as to enable the screen assembly to be tilted relative to the device body for adjusting an angle between the screen assembly and a horizontal plane.

156. The medical device according to claim 155, **characterized in that**:
the first slidable portion is a slide rail, and the second slidable portion is a slidable member; or
the first slidable portion is a slide groove, and the second slidable portion is a slidable member which extends into the slide groove.

157. The medical device according to claim 155 or 156, **characterized in that**, a shape of a rear side of the mounting housing matches with a shape of a front side of the device housing; wherein a space, which enables the screen assembly to be tilted, exists between the mounting housing and the device housing.

158. A monitoring device, which is configured to obtain and process physiological parameter data of a patient acquired by a parameter sensor, and display processed physiological parameter data of the patient, **characterized in that**, the monitoring device comprises:
a main control board, which is configured to obtain the physiological parameter data of the patient acquired by the parameter sensor and process the physiological parameter data;
a device body, which comprises a device housing;
a screen assembly, which comprises a mounting housing and a screen which is arranged at the mounting housing;
a connection assembly, wherein the screen assembly is in movable connection with the device body through the connection assembly, so as to enable the screen assembly to be tilted from a first position to a second position relative to the device body; wherein when the screen assembly is tilted from the first position to the second position, at least a portion of the screen assembly gradually moves away from or close to the device body, wherein tilting of the screen assembly satisfies one of following conditions:
a movement trajectory of a center of mass of the screen assembly is an irregular circular movement;
the screen assembly performs a circular movement around two or more points;
the screen assembly performs a circular movement around two or more axes.

159. The monitoring device according to claim 158, **characterized in that**:
the connection assembly is a cover housing; wherein the cover housing is configured to cover a gap region, which is formed between the device body and the screen assembly due to tilting of the screen assembly relative to the device body; or
the monitoring device further comprises a cover housing; wherein the cover housing is arranged between the device housing and the mounting housing, or is arranged at at least one of the device housing, the mounting housing and the connection assembly, so as to cover a gap region, which is formed between the device body and the screen assembly due to tilting of the screen assembly relative to the device body.

160. The monitoring device according to claim 159, **characterized in that**, at the second position, the cover housing surrounds, and encloses or seals the gap region.

161. The monitoring device according to claim 159, **characterized in that**, at least one of the device housing, the mounting housing and the connection assembly is provided with a mounting portion of the cover housing for mounting the cover housing, when the cover housing is mounted at the mounting portion of the cover housing, the cover housing covers a gap region, which is formed between the device body and the screen assembly due to tilting of the screen assembly relative to the device body.
